# EUROPEAN PATENT SPECIFICATION

(11) **EP 2 379 055 B1**
(45) Date of publication and mention of the grant of the patent: **08.11.2017**
(21) Application number: 09830961.0
(22) Date of filing: 01.12.2009
(51) Int. Cl.: A61K 9/14, A61K 31/519, A61K 9/16

(54) **METHOD OF MAKING SUSTAINED RELEASE MICROPARTICLES**
VERFAHREN ZUR HERSTELLUNG VON MIKROTEILCHEN MIT VERZÖGERTER FREISETZUNG
PROCÉDÉ DE FABRICATION DE MICROPARTICULES À LIBÉRATION PROLONGÉE

(30) Priority: 04.12.2008 US 328136
(43) Date of publication of application: 26.10.2011
(73) Proprietor: Oakwood Laboratories, Llc, Oakwood Village, OH 44146 (US)
(72) Inventor: THANOO, Bagavathikanun, Chithambara, Brecksville OH 44141 (US); JOHNS, Gonto, III, North Olmsted OH 44070 (US); WOO, Byung, Ho, Broadview Heights OH 44147 (US)
(74) Representative: Tostmann, Holger Carl
(86) International application number: PCT/US2009/066212
(87) International publication number: WO 2010/065513

(56) References cited:
- US-A- 5 945 126
- US-A1- 2005 042 294
- US-B1- 6 270 802
- LUAN X ET AL: "Key parameters affecting the initial release (burst) and encapsulation efficiency of peptide-containing poly(lactide-co-glycolide) microparticles", INTERNATIONAL JOURNAL OF PHARMACEUTICS, ELSEVIER BV, NL, vol. 324, no. 2, 6 November 2006 (2006-11-06), pages 168-175, XP025113190, ISSN: 0378-5173, DOI: 10.1016/J.IJPHARM.2006.06.004 [retrieved on 2006-11-06]
- JEYANTHI R ET AL: "Effect of solvent removal technique on the matrix characteristics of polylactide/glycolide microspheres for peptide delivery", JOURNAL OF CONTROLLED RELEASE, ELSEVIER, AMSTERDAM, NL, vol. 38, no. 2, 1 February 1996 (1996-02-01), pages 235-244, XP004037409, ISSN: 0168-3659, DOI: 10.1016/0168-3659(95)00125-5
- YI-YAN YANG ET AL: "Effect of preparation conditions on morphology and release profiles of biodegradable polymeric microspheres containing protein fabricated by double-emulsion method", CHEMICAL ENGINEERING SCIENCE, vol. 55, 1 June 2000 (2000-06-01), pages 2223-2236, XP055178762,

## Description

### BACKGROUND

Microparticles and microspheres formed from various natural and synthetic polymers and resins have become popular delivery vehicles for various active agents such as drugs, diagnostic reagents, and the like. Degradable sustained release microparticles are of particular interest for use in so call "depot" formulations, where delivery of the active agent over an extended period of time is desired.

Optimum compositions of sustained release microparticle formulations must release an effective amount of biologically active agent in a therapeutic amount effective to treat a specific condition in a specific amount of time. Generally, the sustained release compositions include a release profile, including the rate at which the active agent is released from the microparticle into a patient's system. It may be beneficial to modify the release profiles of the sustained release microparticles in a manner including changing the morphology of the microspheres by altering the ratio of the dispersed and external or continuous phases (Luan X, Skupin M, Siepmann J, Bodmeier R, Key parameters affecting the initial release (burst) and encapsulation efficiency of peptide-containing poly(lactide-co-glycolide) microparticles. International J. of Pharmaceutics. 2006; 324: 168-175). Alternatively, the release rate profiles can also be affected by the rate of continuous water-phase addition (Yang YY, Chung TS, Bai XL, Chan WK, Effect of preparation conditions on morphology and release profiles of biodegradable polymeric microspheres containing protein fabricated by double-emulsion method. Chemical Engineering Science. 2000; 55: 2223-2236) that would impact the morphology of the microspheres and further allow control of the release rates for the specific active agents encapsulated within the microparticle. But employing the above processes takes longer duration for the formation of solid microspheres.

### SUMMARY

One embodiment includes a method for forming sustained release microparticles including the steps of forming a dispersed phase with an active agent dissolved with a polymer; mixing the dispersed phase with an aqueous continuous phase to form a
microparticle dispersion having microparticles suspended in the continuous phase; and adding a measured amount of a dilution composition to the microparticle dispersion after the microparticles have been formed; wherein the formed microparticles are sufficiently solid to be filterable using a hollow fiber filter and wherein the amount of the dilution composition is sufficient to alter the release rate for the active agent.

Another embodiment of the invention includes a method for forming sustained release microparticles including the steps of forming a dispersed phase with an active agent and a polymer; mixing the dispersed phase and an aqueous continuous phase to form a microparticle dispersion having microparticles that are sufficiently solid to be filterable using a hollow fiber filter suspended in the continuous phase; and adding a dilution composition to the microparticle dispersion to produce a sustained release microparticle having a release rate
different than a release rate of an untreated sustained release microparticle; wherein the volume of the dilution composition is at least 50% of the volume of the continuous phase.

Yet another embodiment includes a method for controlling a release rate of a sustained release microparticle including the steps of forming a dispersed phase with leuprolide and a polymer, wherein the leuprolide is dissolved in the polymer. The method further includes mixing the dispersed phase with a continuous phase in a mixer to form a microparticle dispersion and adding a measured amount of a dilution composition to the microparticle dispersion after microparticles have been formed, wherein the formed microparticles are sufficiently solid to be filterable using a hollow fiber filter. The amount of a dilution composition is also sufficient to alter the release rate for the leuprolide.

Also disclosed herein is a sustained release composition with an altered release rate having a dispersed phase with a specific active agent and a polymer, the dispersed phase combined with a continuous phase to form a microparticle dispersion, and the microparticle dispersion being exposed to a measured amount of a dilution composition after microparticles are formed; wherein the dilution composition is sufficient to dilute the continuous phase to alter the release rate of the specific agent.

### BRIEF DESCRIPTION OF DRAWINGS

Fig. 1 is a schematic representation of one embodiment of the method of the present invention;
Fig. 2 is a graphic representation of the release properties of microparticles in an in vivo rat study as prepared and described in Tables 3 and 4, according to an embodiment of the present invention;.
Fig. 3 is a graphic representation of the release properties of microparticles in an in vivo rat study with leuprolide microparticles as prepared and described in Tables 3 and 4, according to an embodiment of the present invention;
Fig. 4 is a graphic representation of the release properties of microparticles as prepared and described in Tables 5 and 6; according to an embodiment of the present invention;
Fig. 5 is a graphic representation of the release properties of microparticles as prepared and described in Tables 7 and 8; according to an embodiment of the present invention;
Fig. 6 is a graphic representation of the leuprolide acetate release from microparticles under accelerated in-vitro release conditions as prepared and described in Tables 5 and 6; according to an embodiment of the present invention;
Fig.7 is a graphic representation of the release rate of leuprolide acetate from microparticles under accelerated in-vitro release conditions as prepared and described in Tables 7 and 8; according to an embodiment of the present invention;
Fig. 8 is a graphic representation of serum leuprolide concentration levels in rats that received two as prepared and described in Tables 9 and 10; according to an embodiment of the present invention;
Fig. 9 is a graphic representation of serum leuprolide concentration levels in humans as prepared and described in Tables 9 and 10, according to an embodiment of the present invention; and
Fig. 10 is a graphic representation of release properties of microparticles in an in-vivo study of octreotide in Rats as prepared and described in Table 11; according to an embodiment of the present invention.

### DETAILED DESCRIPTION

Disclosed herein are methods for optimizing the release profiles of biologically active agents encapsulated in sustained release microparticles in warm blooded mammals, including humans. Also disclosed are methods for changing the release rate of the biologically active agent from the microparticles by treating the post-formation microparticles with an added dilution composition as part of a continuous, aseptic process.

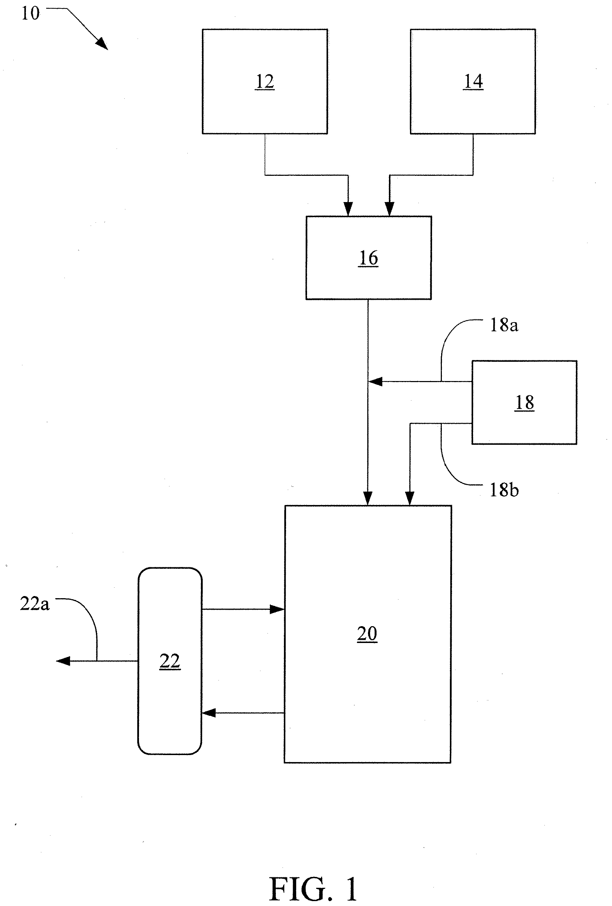

As shown in Fig. 1, one embodiment includes a method 10 for optimizing the initial release rate of an active agent from a sustained release microparticle. In this embodiment, the release profile of leuprolide acetate in a microparticle may by modified by adjusting the concentration of solvents to which the freshly formed microparticles are exposed. The release profiles of the microparticles may be modified while the microparticles remain in an aqueous phase at room temperature without associated steps that may introduce residual solvent impurities or aggregation problems during the steps of drying, heating, or reconstituting with water.

In a sustained release product, the amount of drug initially released from the composition can be important. The amount of drug, or active agent, released upon initial administration is often referred to as an initial burst or release. The amount of initial burst or release is important to consider and to optimize for specific drugs and their intended use.

For example, leuprolide acetate, "leuprolide," is a biologically active agent that is a known agonist analogue of gonadotrophin-releasing hormone (GnRH). GnRH stimulates the production of a luteinizing hormone (LH) and the follicle stimulating hormone (FSH). When released into the bloodstream, these hormones cause the production of testosterone in men and estrogen in women. Therefore in diseases such as prostrate cancer, where controlling the level of testosterone produced has been shown to be an effective treatment, an active agent that would inhibit the production of GnRH, LH, and FSH would be beneficial. In effect, controlling the level of testosterone produced may limit the need for surgical castration.

In therapeutic applications, leuprolide initially releases LH and FSH from the anterior pituitary. However, with continued use, leuprolide causes pituitary desensitization and down-regulation of the testosterone producing hormones. When used in treatment, leuprolide suppresses circulating sex hormone levels in 2 to 4 weeks, after the initial rise. Therefore, in order for leuprolide to be efficacious, a very high initial release rate of the drug from the delivery microparticle may be desirable. According to the Food and Drug Administration, for leuprolide, a high initial release may be required to achieve faster castration. Chemical castration using leuprolide is defined as 95% of the patients having testosterone levels at or below 50 ng/dL (0.5 ng/mL) by 28 days or earlier.

In contrast, octreotide, another active agent, is a somatostatin analog used for long term treatment therapy in acromegalic patients, as well as for patients with malignant carcinoid tumors, vasoactive intestinal peptide tumors, flushing, and diarrhea. Octreotide in serum controls growth hormone (GH) and insulin-like growth factor-1 (IGF-1). A common disadvantage of the use of octreotide in long term depot sustained release formulations is that high peak levels of octreotide can be toxic at certain plasma concentrations. A slower initial burst or release rate would be preferable in this situation.

As shown in Fig. 1, a continuous process 10 for producing lueprolide-containing microparticles may be used to produce the microparticles. The continuous process 10 may generally include combining a dispersed phase 12 and a continuous phase 14. The dispersed phase 12 may include the active agent, a polymer, and suitable solvents. The continuous phase 14 may generally include an aqueous solution. The dispersed phase 12 and the continuous phase 14 may be combined in an in line mixer 16 to form a microparticle dispersion. The microparticle dispersion may generally be comprised of freshly formed microparticles dispersed in an aqueous solution.

For the purposes of this application, the formulation of leuprolide in a sustained release microparticle for the treatment of prostate cancer will be used as an one example of an active agent being released from a polymeric sustained release microparticle. It will be apparent to those of ordinary skill in the art, in view of the disclosure, that the active agent can be any agent for which encapsulation or interspersion within a small polymer body is desired. Further examples of active agents whose release rate can be optimized to improve clinical effectiveness include peptides, ketotifen, thioridazine, olanzapine, risperidone, oxybutynin, octreotide, naltrexone, orntide, or Woc4D, betaqmethasone, dexamethasone, clonidine, verapamil, or pharmaceutically acceptable salts thereof. Of particular interest are luteinizing hormone-releasing hormone agonists (LH-RH agonists) such as leuprolide, triptorelin, goserelin, nafarelin, historelin, and busereline. Also of particular interest are somatostatin analogs such as octreotide, human, salmon and eel calcitonin, growth hormones, growth hormone releasing hormones, growth hormone releasing peptide, parathyroid hormones and related peptides, interferon, erythropoietin, GM-SSF, G-CSF, thymosin, antitrypsin, and chemotherapy drugs, antibiotics and analgesics for regional administration and other agents where adjustment of the release rate can increase the effectiveness of the sustained release formulation.

In order to incorporate the active agent into the dispersed phase 12 it is usually necessary to dissolve the active agent in at least one solvent. Solvents for the active agent will of course vary depending upon the nature of the agent. Typical solvents that may be used in the dispersed phase to dissolve the active agent include, but are not limited to, water, methanol, ethanol, dimethyl sulfoxide (DMSO), dimethyl formamide, dimethyl acetamide, dioxane, tetrahydrofuran (THF), dichloromethane (DCM), ethylene chloride, carbon tetrachloride, chloroform, lower alkyl ethers such diethyl ether and methyl ethyl ether, hexane, cyclohexane, benzene, acetone, ethyl acetate, methyl ethyl ketone, acetic acid, or mixtures thereof. Additionally, an acid such as glacial acetic acid, lactic acid, or fatty acids or acrylic acid may be used in the process to help improve the solubility and encapsulation of the active agent in the polymer. Selection of suitable solvents for a given system will be within the skill in the art in view of the instant disclosure.

The active agent is then combined with a polymer to form the dispersed phase 12. Examples of polymers known to those of ordinary skill in the art, and useful in one embodiment, may be found in, for example, in U.S. Pat. Nos. 4,818,542, 4,767,628, 3,773,919, 3,755,558 and 5,407,609. In selecting a particularly desirable polymer for a given system, numerous factors can be considered for purposes of producing a product having the desired clinical characteristics such as biodegradability (e.g., release profile) and biocompatibility. Once one of ordinary skill in the art has selected a group of polymers that will provide the desired clinical characteristics, then the polymers can be evaluated for desirable characteristics that will optimize the manufacturing process. For example, in some instances, it may be possible to select a polymer that will interact with the active agent in a manner that will facilitate the processing of the microparticles, enhance drug load, enhance solvent removal from the dispersed phase or inhibit drug migration from the dispersed phase into the continuous phase.

Examples of suitable polymers are homopolymers of lactic acid or copolymers of lactic acid and glycolic acid, or poly(lactide-co-glycolide), "PLGA" polymers. The ratio of lactic acid residues to glycolic acid residues can vary, and will typically range from 25:75 to 85:15, although even a 10% glycolide could find use since high lactide content results in lower viscosity and higher solubility. Preferred copolymers comprise at least about 50% lactic acid residues, such as 50:50, 75:25, or 85:15 polymers. Poly(lactide-co-glycolide) copolymers are commercially available from a number of sources and can be readily prepared by conventional synthetic routes. Boeringer Inglehiem produces suitable polymers under the designations R 202H, RG 502, RG 502H, RG 503, RG 503H, RG 752, RG 752H, RG 756 and others. LH-RH microparticles with R202H, RG752H, or RG503H may be used in the dispersed phase of one embodiment,. Selection of a suitable polymer for a given system would be apparent to those of ordinary skill in the art in view of this disclosure.

One consideration in selecting a preferred polymer is the hydrophilicity/hydrophobicity of the polymer. Both polymers and active agents may be hydrophobic or hydrophilic. Where possible it is desirable to select a hydrophilic polymer for use with a hydrophilic active agent, and a hydrophobic polymer for use with a hydrophobic active agent. In the preferred microparticles, an ionic interaction between the drug and the hydrophilic carboxyl groups of the polymer is believed to enhance the drug load. In general, however, since hydrophilic drugs are soluble in water, if there is no affinity between the polymer and drug, or solidification is not sufficiently fast, drug load may decrease. It is also possible to use a hydrophilic drug in a hydrophobic polymer.

In selecting a particular polymer, the effect of the hydrophobicity/hydrophilicity of the polymer on the residual solvent in the system should also be considered. A hydrophilic polymer can be expected to yield low residual solvent with a hydrophilic drug, such as a hydrophilic peptide. In the case of the leuprolide microparticles, the drug has a tendency to help eliminate hydrophobic solvent from the dispersed phase droplets quickly and efficiently. In addition, it has been observed that a greater drug load tends to correlate to lower residual solvent concentrations. Thus, in some systems, there is an indirect benefit with lower residual solvent when incorporating hydrophilic drugs in hydrophilic polymers. However, since there are other influencing factors on residual solvent other than hydrophilicity, this effect may not uniformly apply to non-peptide drugs. Nevertheless, it should follow that active agents that enhance the elimination of solvent from the dispersed phase droplet, without concomitant drug loss, yield superior products.

Another consideration is molecular weight of the polymer. While the molecular weight of the polymers will obviously impact on the product characteristics such as release rate, release profile and the like, it can also impact the process of producing the microparticles. Higher molecular weight polymers are typically associated with a more viscous dispersed phase, resulting in larger particles or increased difficulties in obtaining small particles and, in some instances, increased residual solvent. By contrast, lower molecular weight polymers are typically associated with slower solidification because the polymer tends to be more soluble. In the preferred system, higher residual solvent, higher drug loading and enhanced incorporation efficiency has been found to result from the use of higher molecular weight polymers. One advantage of the inventive process is its ability to form good, small, low residual solvent microparticles with high molecular weight polymers and, hence, viscous dispersed phases. Of course, the particular selection will also depend upon the desired product characteristics. For example, the higher the molecular weight, the longer the degradation time in the body and the longer the duration of drug release.

Still further, the particular polymer concentration employed can effect the system, not only from a product morphology standpoint, but also from a processing standpoint. An increase in polymer concentration tends to be associated with a higher drug load because a viscous dispersed phase needs to eliminate less solvent for solidification. An increased solidification rate tends to cause higher drug retention. Moreover, a viscous dispersed phase leads to less drug diffusion into the continuous phase during solidification. In some systems this may also result in higher residual solvent. In the preferred embodiments, polymer concentration in the dispersed phase will be from about 5 to about 40%, and still more preferably from about 8 to about 30%.

Solvents for the polymer will be appropriately selected depending upon a number of factors, including the nature of the polymer, the active agent, toxicity, compatibility with other solvents in the system and even the use to which the microparticle will be put. Thus, in addition to dissolving the polymer, the solvent must be immiscible with the continuous phase in order to form droplets, highly volatile for optimum evaporation efficiency, and desirably non-flammable for safety reasons. Solvents suitable for the preferred poly(lactic) or poly(lactide-co-glycolide) polymers include methylene chloride, chloroform, ethyl acetate, substituted pyrrolidone and the like. In some instances, the solvent for the active agent will be the same as the solvent for the polymer. Some drugs, typically diagnostic agents such as radioactive inorganic salts used in imaging analysis, are not soluble or only slightly soluble in organic solvents. In these instances, a fine, sub-sub micron size powder can be directly suspended in the polymer solution to form microparticles. Although resort to this will be rare in drug delivery, it may prove useful with diagnostic agents. Selection of other solvents useful in accordance with the process of the invention will be within the skill in the art in view of the instant disclosure.

In this embodiment, the leuprolide containing poly(lactide) or poly(lactide-co-glycolide) microparticles may be prepared by dissolving the leuprolide active agent in the R202H polymer with methylene chloride, methanol, and glacial acetic acid to form the dispersed phase 12. The dispersed phase may be a true, homogeneous solution. Alternatively, separate solutions of polymer and active agent can be prepared, each in its own solvent, and subsequently mixed to form the dispersed phase 12. In some instances, due to the nature of the active agent and/or polymer, the dispersed phase 12 must be formed as an emulsion. For example, when a given proteinaceous drug is dissolved in a suitable active agent solvent, the resulting solution may be completely immiscible with a solution of the polymer in a particular polymer solvent. In order to provide a relatively homogeneous dispersed phase 12 in which the drug and polymer are relatively uniformly interspersed, the drug and drug solvent may be emulsified with the polymer and polymer solvent to form a dispersed phase emulsion. Upon introduction of the dispersed phase 12 into the continuous phase 14 a water-oil-water, or w/o/w, emulsion may be formed. In still other systems, the dispersed phase 12 can be prepared by forming a direct suspension of the active agent in a polymer solution.

In accordance with the inventive process described below, the dispersed phase 12 may be mixed with the continuous phase 14 in order to form a microparticle dispersion having droplets or inclusions of the dispersed phase 12 dispersed within the continuous phase 14. As used herein the term dispersed is intended in its broadest sense as meaning discrete regions of dispersed phase interspersed within the continuous phase. The noted inclusions will typically occur as generally spherical droplets, but may in some instances be irregular inclusions due to particular emulsification conditions. Any suitable medium in which the dispersed phase 12 will form droplets or inclusions may be used as a continuous phase 14, with those that provide a maximum solvent sink for the dispersed phase 12 solvent being especially desirable.

Frequently, the continuous phase 14 will also contain surfactant, stabilizers, salts or other additives that modify or effect the emulsification process. Typical surfactants include sodium dodecyl sulphate, dioctyl sodium sulfo succinate, span, polysorbate 80, tween 80, pluronics and the like. Particular stabilizers include talc, PVA and colloidal magnesium hydroxide. Viscosity boosters include polyacrylamide, carboxymethyl cellulose, hydroxymethyl cellulose, methyl cellulose and the like. Buffer salts can be used as drug stabilizers and even common salt can be used to help prevent migration of the active agent into the continuous phase 14. One problem associated with salt saturation of the continuous phase 14 is that PVA and other stabilizers may have a tendency to precipitate as solids from the continuous phase. In such instances a particulate stabilizer might be used. Suitable salts, such as sodium chloride, sodium sulfate and the like, and other additives would be apparent to those of ordinary skill in the art in view of the instant disclosure.

In one embodiment, the continuous phase 14 includes from 100-50% water. The aqueous continuous phase 14 may include a stabilizer. A preferred stabilizer is polyvinyl alcohol (PVA) in an amount of from about 0.1% to about 5.0%. More specifically, PVA may be present in an amount of about 0.35%. Other stabilizers suitable for use in the continuous phase 14 would be apparent to those of ordinary skill in the art in view of the instant disclosure.

The selection of particular polymers, solvents and continuous phases will of course vary depending on the active agent and the desired product characteristics. Once the desired product characteristics, such as clinical application, release profile and the like are established, there may nevertheless be some latitude in selecting polymers, solvents and continuous phases to facilitate the production process.

Once the dispersed phase 12 and continuous phase 14 are combined, the freshly formed microparticles may be continuously transferred from the mixer 16 to a first vessel 20. The microparticles are generally sufficiently solid and filterable, such that they are suitable for the continuous processing of microparticles using hollow fiber filter, as disclosed in US patents 6,270,802 and 6,361,798. This method is distinguished from a general method of forming microparticles wherein the mixture of the dispersed phase and the continuous phase do not form discrete microparticles quickly. For example combining the dispersed phase and continuous phase may result only in the formation of
an emulsion containing soft microspheres. Additional processing steps may be required to harden them sufficiently. (Jeyanthi R, Thanoo BC, Metha RC, DeLuca PP, Effect of solvent removal technique on the matrix characteristics of polylactide/glycolide microspheres for peptide delivery. J of Controlled Release. 1996; 38: 235-244).

As opposed to a method where the microparticles require further formation procedures, the microparticles formed in this embodiment solidify within about 20 seconds, more preferably less than about 10 seconds, and still more preferably less than about 5 seconds. In a leuprolide-polymer embodiment, the microparticles are solidified in less than about 3 seconds. It is observed that in the instant embodiment, even though solidification of microparticles is virtually instantaneous upon addition to the continuous phase, the formed microparticles are nevertheless susceptible to give up additional residual solvent into the continuous phase.

Drug load, in the case of the preferred leuprolide/polymer microparticles, is targeted about 12%-in the range of 10 to 21% based on the total solid. In practice, drug loads on the order of about 9 to 17about 17% can be obtained. Of course, the nature of the drug, the desired release profile, the nature of the polymer and, of course, processing can all effect the desired and actual drug load. In the typical case, drug loads on the order of 5% to 20% based on the combined weight of drug and polymer are desired and achievable with the process of the invention. Typical encapsulation efficiency is around 75%.

Advantageously, once the desired drug load is obtained, and the parameters of feed rate, temperature, etc., are determined, scaling up to larger batches, including production level batches, becomes a simple matter of running the process longer. No additional feed tubes, emulsifiers, impellers or the like are necessary to produce a larger number of microparticles having the desired characteristics. Moreover, the microparticles produced during the continuous process of the invention are exceptionally uniform in terms of size distribution, agent load and the like, regardless of when during the process they were produced.

In one embodiment, it has been found that the general process of forming microparticles, as described above, may be altered to change the release properties or profiles of the microparticle batches by varying the solvent exposure of the microparticles. Lower levels of solvent exposure post formation can be accomplished by adding a dilution composition 18 to the freshly formed microparticles. The dilution composition 18 may be water or a polyvinyl alcohol solution. The addition of a measured amount of dilution composition 18 after the microparticles are formed has surprisingly been found to alter the release rate of the freshly formed microparticles.

As shown in Fig. 1, after the microparticles are formed and dispersed within the continuous phase, a dilution composition 18 may be added. The dilution composition, as shown in Fig. 1 at 18a, may be added to the microparticle dispersion as it is transferred to a first vessel 20. Adding the dilution composition to the microparticle dispersion as it is being transferred to a first vessel 20 may allow the microparticle dispersion and the dilution composition to be thoroughly mixed before it reaches the vessel 20 and is filtered using a hollow fiber filter 22.

Alternatively, as shown at 18b, the dilution composition 18 may be transferred to the vessel 20 before the microparticle dispersion or at the same time as the dispersion. Once in the first vessel, the mixture of microparticles and dilution composition may be continuously stirred.

The amount of dilution composition 18 added to the microparticle dispersion should be sufficient to adjust the release profile of the final formulation for injection into mammals. The dilution composition 18 should not adversely or drastically change the solubility of the active agent encapsulated in the microparticles, the particle size of the microparticles, or the encapsulation efficiency of the active agent in each of the batches. A predetermined solvent exposure level, correlating to a predetermined amount of dilution composition 18 to be added, is calculated for a desired release rate. The specific release rate is then obtained through the addition of the dilution composition 18.

In one embodiment, to obtain leuprolide microparticles with the desired release profile, dilution composition 18 is added until the set concentration of solvent in the continuous phase is less than 5000 ppm. By adding a dilution composition 18 in a defined amount and time, the release profile can be adjusted as part of a continuous process in a predictable and controllable manner.

The amount of dilution composition added to the formed microparticles may be at least about 50% of the original volume of the continuous phase 14. In one embodiment, the decreased exposure to solvent in the aqueous solution may increase the release rate of the formed leuprolide microparticles. Controlling the release rate for different active agents, however, may require different steps to achieve the desired release profile. The process is easily adaptable to accommodate the use of different active agents with different release profiles.

As the microparticle dispersion from mixer 16 and the dilution composition 18 are combined in the first vessel 20, the microparticle/dilution composition mixture is filtered through a hollow fiber filter 22. The microparticles are returned to the first vessel 20 while the waste material is discarded into a separate vessel via line 22a. As the microparticles are returned to vessel 20 they are continuously mixed with new microparticle/dilution composition mixture and cycled through the filter.

The microparticles after having been processed with the necessary composition and time to achieve the desired release rate may be then filtered to remove any remaining aqueous solution and washed with water to remove the process by-products. Once the microparticles have been formed, it will readily apparent to one skilled in the art that there are many final adjustments that may be made to prepare the microparticles for a filling vessel to prepare them for patient administration.

The process according to the invention will now be exemplified by the following non-limiting examples, with reference to accompanying drawings.

### EXAMPLES

The following examples are set forth to provide those of ordinary skill in the art with a detailed description of how the methods claimed herein are carried out and evaluated, and are not intended to limit the scope of what the inventors regard as their invention. Unless indicated otherwise, parts are by weight and temperature is in °C.

Molecular weights are reported as weight average (Mw) molecular weight or number average (Mn) molecular weight and were determined by gel permeation chromatography using (GPC) using polystyrene (PS) molecular weight standards. Example 1 provides a general illustration of one embodiment of the method. Poly(d,l-lactide), R202H is commercially available from Beohringer Ingelheim.

### Example 1

Leuprolide acetate microparticles were prepared as described Table 1 below. The microparticles were formed by mixing a dispersed phase and continuous phase to form approximately 7L of a microparticle dispersion. Generally, the dispersed phase may be a solution of leuprolide acetate (L.A.), poly(d,l-lactide) (R202H), and solvents methylene chloride (DCM), methanol (MeOH), and glacial acetic acid (A.Acid), according to the parameters described in Table 1. R202H will provide sustained release of leuprolide for 3 to 4 months based on in-vivo studies in warm blooded animals and humans. The continuous phase may be prepared using a 0.0035 g/g polyvinyl alcohol solution.

The continuous phase and dispersed phase were simultaneously added to an in-line Silverson mixer at a flow rate of 2 L/min and 38 g/minute, respectively. The mixing speed in the mixer was 7000 rpm and the microparticle formation duration was 3.5 minutes. This microparticles dispersion was continuously delivered into a vessel containing approximately 40L of a dilution phase.

The dilution phase may also contain a 0.0035 g/g polyvinyl alcohol aqueous solution, however the dilution phase is spiked with solvents as shown in Table 1. The amount of methylene chloride in the dilution phase was varied in each batch, specifically, from about 5000 ppm for microparticle batch GG090303 to about 8500 ppm for microparticle batch GG091003. The corresponding duration for the solvent exposure in the reactor vessel with the solvent spiked dilution phase was constant at 100 minutes for all four microparticle batches.

After solvent exposure, the microparticles suspended in the dilution phase was transferred to a solvent removal vessel (SRV). All of the microparticle batches were washed using an ambient water wash, with two volume changes, for 40 minutes and an air sweep of 80 SLPM; a hot water wash (39°C), with 5 volume changes, for 100 minutes with an air sweep of 80 SLPM; and were cooled, with a 1.5 volume exchange, for 30 minutes with an air sweep at 80 SLPM. The air sweep was introduced to aid the head space solvent removal during the wash process. Microparticles were filtered on a filter membrane and freeze dried under a vacuum.

Table 1 includes both the concentration of the active agent, polymer, and solvents used to prepare the dispersed phase, as well as the concentration and actual amounts of each solvent used for the preparation of the dilution phase.

**Table 1**

| Batch | GG090303 | GC040104 | GC040504 | GG091003 |
|---|---|---|---|---|
| L.A._{DP}conc., g/g | 0.0542 | 0.0548 | 0.0546 | 0.0542 |
| L.A. _{DP} wt., g | 8.5636 | 8.66 | 8.6277 | 8.56 |
| R202H_{DP} conc., g/g | 0.2483 | 0.2482 | 0.2488 | 0.2484 |
| R202H_{DP} wt., g | 39.2 | 39.26 | 39.31 | 39.20 |
| DCM_{DP} conc., g/g | 0.5277 | 0.5282 | 0.5274 | 0.5278 |
| DCM_{DP} wt., g | 83.3 | 83.54 | 83.35 | 83.30 |
| MeOH_{DP} conc., g/g | 0.1656 | 0.1651 | 0.1653 | 0.1655 |
| MeOH_{DP} wt., g | 26.1484 | 26.11 | 26.1282 | 26.12 |
| A. Acid_{DP} conc., gg | 0.0041 | 0.0037 | 0.0039 | 0.0040 |
| A. Acid_{DP} wt., g | 0.6467 | 0.59 | 0.6153 | 0.63 |
| PVA_{CP} conc., g/g | 0.0035 | 0.0035 | 0.0035 | 0.0035 |
| PVA_{Dil.P} conc., g/g | 0.0035 | 0.0035 | 0.0035 | 0.0035 |
| PVA _{Dil.P} wt., g | 140 | 140 | 140 | 140 |
| DCM_{Dil.P} conc., ppm | 5000 | 6038 | 7008 | 8495 |
| DCM_{Dil.P} wt., g | 200 | 241.5 | 280.3 | 340 |
| MeOH_{Dil.P} conc., ppm | 1575 | 1928 | 2200 | 2675 |
| MeOH_{Dil.P} wt., g | 63 | 77.1 | 88.0 | 107 |
| A. Acid_{Dil.P} conc. ppm | 38 | 47 | 55 | 67 |
| A. Acid_{Dil.P} wt. g | 1.5 | 1.88 | 2.20 | 2.60 |

Table 2 details the properties of the microparticles, including particle size, drug release under accelerated release conditions, and drug release under physiological conditions formed by following the preparation parameters in Table 1. The drug load in the microparticle is delineated as a percentage of the active drug in the microparticle. This was determined by dissolving the microparticle in solvent (ex. dimethyl sulfoxide) and adding phosphate buffer to the solution of microparticles. The filtered solution is then assayed by HPLC against calibration standards.

The particle size distribution (Part. Size Dist.) was determined for the suspension of microparticles in water containing surfactant or diluents. This was determined by laser light scattering. The particle size distribution is reported by volume distribution (CVF).

Drug release rate calculations were performed in a phosphate buffer solution (PBS) to determine the release rate of leuprolide under physiological conditions (D.R. (Phys.)). The microparticles formulated as in Table 1 were suspended in PBS in a screw capped glass tube at a temperature of 37°C. At least 80% of the release media was replaced with fresh media at sample points. Released media was assayed by HPLC for drug concentration and calculated for the percentage release.

The release condition can be appropriately manipulated to accelerate the release rate that was achieved in-vivo or by the release in PBS. For leuprolide microparticles the release media used was 0.1 M citrate phosphate buffer and the temperature was 55°C. The release was performed in a shaking water batch, shaking at 170 revolutions per minute. The sample was assayed at each time point for the released drug to find the release rate under accelerated conditions (D.R.(Acc.)).

**Table 2**

| Batch | GG090303 | GC040104 | GC040504 | GG091003 |
|---|---|---|---|---|
| Drug Load, % | 13.71±0.18 | 13.93±0.14 | 13.17±0.18 | 10.37±0.26 |
| Part. Size Dist., | 4.4 | 3.06 | 3.16 | 5.9 |
| 10% CVF Under, micron | | | | |
| Part. Size Dist., | 14.1 | 9.93 | 10.7 | 16.1 |
| 25% CVF Under, micron | | | | |
| Part. Size Dist., | 26.3 | 26.9 | 26.2 | 28.1 |
| 50% CVF Under, micron | | | | |
| Part. Size Dist., | 38.2 | 40.4 | 40.3 | 39.2 |
| 75% CVF Under, micron | | | | |
| Part. Size Dist., | 48.2 | 51.6 | 51.1 | 48.7 |
| 90% CVF Under, micron | | | | |
| D. R. (Phys.), 1 Day | 0.90±0.03 | 0.63±0.01 | 0.25±0.00 | 0.48±0.01 |
| D. R. (Phys.), 7 Days | 1.79±0.18 | 0.88±0.04 | 0.32±0.01 | 0.70±0.04 |
| D. R. (Phys.), 14 Days | 4.30±0.17 | 2.26±0.12 | 0.95±0.01 | 1.13±0.06 |
| D. R. (Phys.), 21 Days | 9.01±0.27 | 5.09±0.23 | 2.64±0.04 | 1.98±0.08 |
| D. R. (Phys.), 28 Days | 24.98±0.47 | 15.79±0.59 | 13.13±0.38 | 10.08±1.42 |
| D. R. (Phys.), 35 Days | 41.47±0.26 | 30.57±0.89 | 30.50±0.60 | 20.40±2.60 |
| D. R. (Phys.), 42 Days | 50.76±2.29 | 44.01±0.82 | 43.60±0.71 | 29.52±2.57 |
| D. R. (Phys.), 49 Days | 55.61±3.68 | 51.14±1.19 | 49.50±1.12 | 35.37±2.03 |
| D. R. (Phys.), 56 Days | 57.24±5.14 | 55.16±1.17 | 52.38±1.72 | 39.08±1.84 |
| D. R. (Phys.), 63 Days | 59.43±5.54 | 57.90±1.30 | 54.66±1.97 | 42.00±1.76 |
| D. R. (Acc.), 1 Hour | 20.4±0.3 | 18.3±0.9 | 12.6±1.4 | 1.3±0.2 |
| D. R. (Acc.), 5 Hours | 39.7±0.4 | 37.3±0.2 | 30.8±0.5 | 8.7±0.7 |
| D. R. (Acc.), 25 Hours | 60.0±0.6 | 59.6±0.4 | 54.1±0.2 | 33.6±0.8 |
| D. R. (Acc.), 48 Hours | 73.2±0.5 | 74.6±0.3 | 70.4±0.5 | 55.9±0.6 |
| D. R. (Acc.), 72 Hours | 81.9±0.6 | 83.6±0.6 | 80.9±0.7 | 72.6±1.0 |
| D. R. (Acc.), 96 Hours | 88.2±0.7 | 90.4±0.7 | 88.1±0.0 | 83.2±0.7 |

Similar particle size distribution and drug load were seen in the microparticles formed according to the preparation steps described above and in Table 1. However, the microparticle batch exposed to the highest exposure level of methylene chloride solvent showed a slightly reduced drug load.

More significantly, the drug release rate of leuprolide varied by the batches in both the accelerated drug release test (Citrate Phosphate buffer at 55"C) and drug release under physiological conditions (Phosphate Buffer Solution, pH 7.4, 37°C). Drug release under the accelerated test conditions provides a quick comparison for the differences in release rates by batch. Drug release under physiological conditions takes place over a longer test period up to and beyond 60 days to simulate the actual duration of the sustained release formulation in clinical applications. Microparticles from batch GG091003, exposed to the highest level of solvent (8500 ppm) consistently had the slowest release rate. Notably, the difference in release rate is more during the initial time points. Generally, microparticle batches made with leuprolide acetate exposed to higher solvent concentrations showed reduced release rates. Therefore, in order to increase the release rate for microparticles containing leuprolide, it is desirable to decrease the solvent exposure to the microparticles after they are formed.

### Example 2

Leuprolide acetate microparticles were prepared as described in Table 3 below. The dispersed phase of the microparticle batches were prepared using consistent composition of leuprolide acetate (L.A.), methylene chloride, methanol, and glacial acetic acid. Two microparticles batches, 0C3542 and 0H8340, were prepared having 0.7 kg to 0.4 kg, respectively, of solid microparticles in the suspension by using a polymer lot of R202H with a molecular weight of 14 kDa. Three microparticle batches, 0D4022, 0E5103, and 0S1723, were prepared having 0.1 kg, 0.4 kg, and 0.4 kg, respectively, solid microparticles in the suspension by using a polymer lot of R202H with a molecular weight of 18 kDa. Each batch was prepared using similar continuous phase formulations and similar preparation parameters. However, the microparticle formation times were varied by varying the batch size, i.e. 0.7 kg as opposed to 0.4 kg of microparticles in suspension, and the time the respective batches of suspension were exposed to the solvent containing continuous phase by varying the flow rate of the dispersed phase into the reactor vessel.

Specifically, the microparticles were formed by an oil in water process by adding the respective dispersed phases to a continuous phase of a 0.35% polyvinyl alcohol solution in water. The mixing speed of the in-line Silverson mixer was 7000 rpm. Upon microparticle formation, the concentration of solvents in the continuous phase of the dispersion was approximately 7200 ppm of methylene chloride and 1600 ppm of methanol.

As After formed in the in-line mixer, the microparticle batches were continuously transferred to a solvent removal vessel. The microparticle formation time was about 40 minutes for the 0.4 Kg batches while the microparticle formation time was about 70 min for 0.7 Kg batches. After 20 minutes of microparticle formation, combining the dispersed phase to the continuous phase at set flow rates, the solvent removal vessel reached the suspension volume of 40L. The suspension in the solvent removal vessel was then re-circulated through a hollow fiber membrane filter (closed system) and permeate was removed at the same rate of suspension feed (2 L/min) to the solvent removal vessel. Thus, the solvent level in the continuous phase remained constant for 40 min for 0.4 Kg batch and 70 min for 0.7 Kg batch.

The microparticle batches were washed using an ambient water wash with two volume changes for 40 minutes and an air sweep of 80 SLPM, a hot water wash (39°C) with 5 volume changes for 100 minutes with an air sweep of 80 SLPM, and were cooled at 1.5 volume exchange of 30 minutes with an air sweep at 80 SLPM. The air sweep was introduced to aid the head space solvent removal during the wash process.

**Table 3**

| Batch | 0C3542 | 0H8340 | 0D4022 | 0E5103 | 0S1723 |
|---|---|---|---|---|---|
| Polymer Intrinsic Viscosity (IV), dL/g | 0.18 | | 0.21 | | |
| R202H M_{w}, kDa | 14 | 14 | 18 | 18 | 18 |
| Batch Size, Kg | 0.7 | 0.4 | 0.7 | 0.4 | 0.4 |
| L.A._{DP} conc., g/g | 0.051 | 0.51 | 0.052 | 0.051 | 0.052 |
| L.A. _{DP} wt., g | 121.4 | 81.57 | 122.5 | 71.5 | 74.0 |
| R202H _{DP} conc., g/g | 0.249 | 0.249 | 0.249 | 0.250 | 0.250 |
| R202H _{DP} wt., g | 588.1 | 400.9 | 588.1 | 352.8 | 352.8 |
| DCM _{DP} conc., g/g | 0.529 | 0.530 | 0.530 | 0.532 | 0.532 |
| DCM _{DP} wt., g | 1250 | 851.9 | 1250.5 | 749.7 | 749.7 |
| MeOH _{DP} conc., g/g | 0.249 | 0.249 | 0.165 | 0.167 | 0.166 |
| MeOH _{DP} wt., g | 392.7 | 267.73 | 390.4 | 235.8 | 233.7 |
| A.Acid _{DP} conc., g/g | 0.004 | 0.004 | 0.004 | 0.004 | 0.004 |
| A.Acid _{DP} wt., g | 9.90 | 6.7 | 10.0 | 5.98 | 5.96 |
| DP Flow Rate, g/min | 34±4 | 34±4 | 34±4 | 34± | 34±4 |

The microparticle properties of the batches formed in Table 3 are shown below in Table 4.

**Table 4**

| Batch | 0C3542 | 0H8340 | 0D4022 | 0E5103 | 0S1723 |
|---|---|---|---|---|---|
| R202 M_{w}, kDa | 14 | 14 | 18 | 18 | 18 |
| Solvent Exposure Duration, min | 70 | 40 | 70 | 40 | 40 |
| Drug Load, % | 11.0 | 13.4 | 12.0 | 13.9 | 14.5 |
| Part. Size Dist., | 4 | 3 | 3 | 3 | 3 |
| 10% CVF Under, micron | | | | | |
| Part. Size Dist., | 10 | 12 | 11 | 11 | 12 |
| 25% CVF Under, micron | | | | | |
| Part. Size Dist., | 23 | 25 | 25 | 26 | 28 |
| 50% CVF Under, micron | | | | | |
| Particle Size Dist., | 34 | 36 | 36 | 39 | 41 |
| 75% CVF Under, micron | | | | | |
| Part. Size Dist., | 42 | 45 | 45 | 50 | 51 |
| 90% CVF Under, micron | | | | | |
| D. R. (Phys.), 1 Day | 0.4 | 0.2 | 0.3 | 0.3 | 0.3 |
| D. R. (Phys.), 7 Days | 0.6 | 1.8 | 0.5 | 0.7 | 0.9 |
| D. R. (Phys.), 14 Days | 2.1 | 4.9 | 0.9 | 2.5 | 3.0 |
| D. R. (Phys.), 21 Days | 6.2 | 19.2 | 1.6 | 4.2 | 4.8 |
| D. R. (Phys.), 28 Days | 14.7 | 32.4 | 9.3 | 17.2 | 14.6 |
| D. R. (Acc.), 1 Hour | 3.0 ± 0.3 | 14.9 ± 0.6 | 7.7 ± 0.5 | 15.6 ± 0.8 | 18.4 ± 0.9 |
| D. R. (Acc.), 5 Hours | 20.6 ± 0.1 | 36.0 ± 0.3 | 24.3 ± 0.2 | 36.2 ± 0.5 | 38.8 ± 0.7 |
| D. R. (Acc.), 25 Hours | 52.2 ± 1.1 | 61.2 ± 0.8 | 48.1 ± 0.0 | 58.6 ± 0.6 | 59.2 ± 0.3 |
| D. R. (Acc.), 48 Hours | 71.4 ± 1.2 | 75.1 ± 0.5 | 66.2 ± 0.0 | 72.5 ± 0.7 | 72.2 ± 0.2 |
| D. R. (Acc.), 72 Hours | 82.3 ± 1.5 | 83.3 ± 0.9 | 78.3 ± 0.2 | 81.0 ± 0.6 | 80.0 ± 0.7 |
| D. R. (Acc.), 96 Hours | 87.9 ± 1.8 | 88.6 ± 1.1 | 85.3 ± 0.3 | 86.4 ± 0.6 | 85.2 ± 0.7 |

The particle size is comparable for the batches. The drug release properties were tested under physiological and accelerated conditions, as described above with regard to Example 1. Results showed reduced initial release for the microparticles OC3542 and 0D4022 that had longer solvent exposure duration, i.e. increase solvent exposure. The microparticles produced from batch OC3542, produced from low molecular weight polymer (M_{w} = 14kDa), had the lowest release rate over most time intervals under both physiological and accelerated test conditions. Therefore, upon high solvent exposure, the lower the molecular weight of the polymer, the lower the initial release rate of leuprolide.

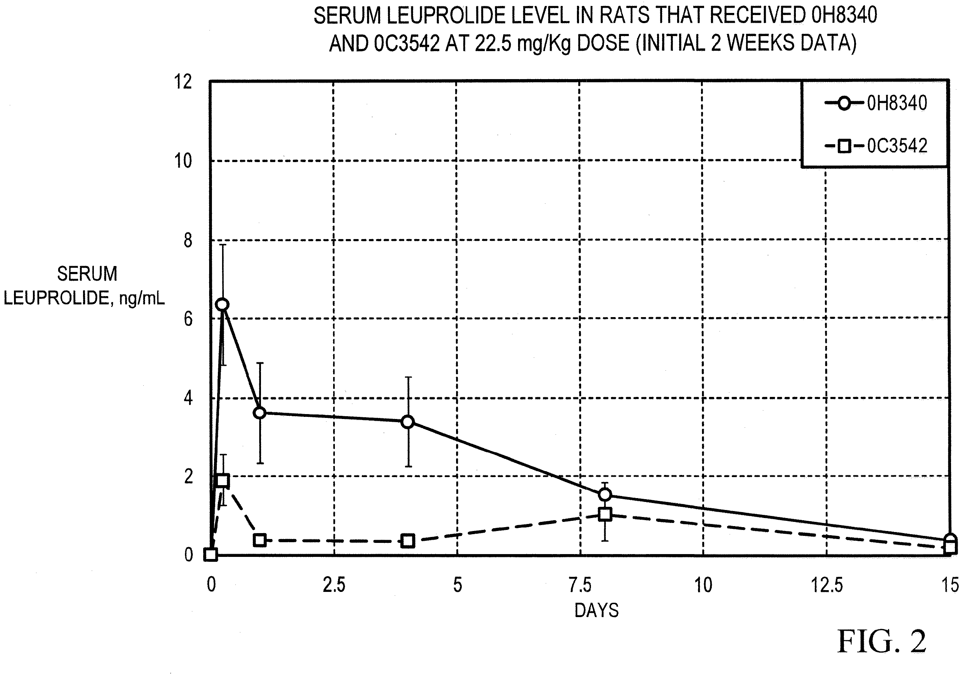

The microparticles prepared as in Tables 3 and 4 were injected as a suspension at 9 mg leuprolide acetate per kg weight of rats. Fig. 2 compares the serum leuprolide in rats that received similar doses of Batch 0H8340 (40 minute solvent exposure, 14 kDa) and Batch 0C3542 (70 minute solvent exposure, 14 kDa). Although both materials were made with polymer having a molecular weight of 14kDa, 0C3542 was made in 0.7 kg batch size with 70 minutes of solvent exposure, while 0H8340 was made from a 0.4 kg batch size and 40 minutes of solvent exposure. This study shows that 0C3542 produced lower drug level in serum initially due to slower early release of drug from the microparticles. The second phase of the release after approximately three weeks produced comparable drug levels. This confirms the lower initial release from the microparticles from batch 0C3542 that underwent longer solvent exposure, with a larger batch size.

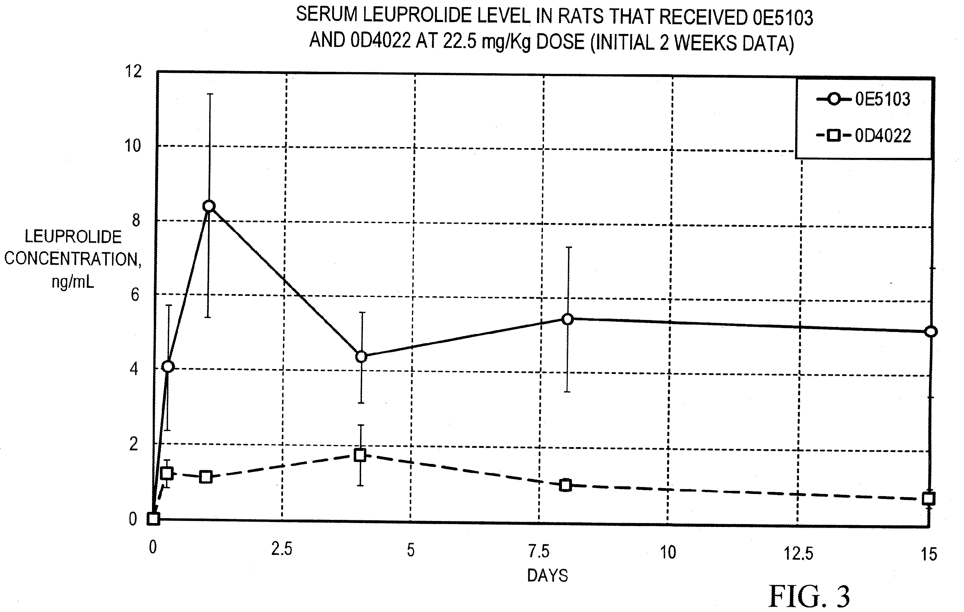

Fig. 3 compares the serum leuprolide in rats that received similar doses of Batch 0D4022 (70 minute solvent exposure, 18 kDa) and 0E5103 (40 minute solvent exposure, 18 kDa). Although both materials were made with polymer having a molecular weight of 18 kDa, 0D4022 was made in 0.7 kg batch size with 70 minutes of solvent exposure, while 0E5103 was made from a 0.4 kg batch size and 40 minutes of solvent exposure. Again, the batch with the longer duration of solvent exposure, 0D4022, produced lower drug levels initially in serum confirming the slower release during the early stage of exposure.

### Example 3

Leuprolide microparticles were made with a polymer having an 0.206 dl/g intrinsic viscosity and a molecular weight of 18 kDa. The microparticle batches were prepared using a continuous processing procedure at 10 g scale. Preparation parameters such as dispersed phase composition, continuous phase composition, disperse phase flow rate, continuous phase flow rate, and the mixer rotations per minute were kept same for all the batches.

Specifically, the microparticles were formed by an oil in water process by mixing the respective dispersed phase and the continuous phase. The continuous phase was comprised of 0.35% polyvinyl alcohol solution in water. The mixing speed of the in-line Silverson mixer was 7000 rpm.

The microparticle dispersion from the mixer is transferred to a solvent removal vessel. In each batch, an amount of a dilution composition was added to the solvent removal vessel. The dilution composition in each batch was water. The dilution composition was added to the solvent removal vessel simultaneously while the dispersion from the in line mixer was delivered into the solvent removal vessel. Specifically, batch GC071304 was prepared without an increased dilution composition, batch GC060404 was prepared with a dilution composition equivalent to 50% of the volume of the continuous phase. Batch GC060404 was delivered from Silverton mixer at a rate of approximately 2 Kg/min, the suspension was diluted with room temperature water (22 to 25°C) with simultaneous flow at about 1 Kg/min.

For batch GC060304, the dilution composition added was equivalent to about 100% of the volume of the continuous phase, in which the batch was delivered from the mixer at a rate of 2 kg/min and the dilution composition was added simultaneously at the rate of 2 kg/min. For batch GC061104, the dilution composition was added at the rate of 4 kg/min, while the suspension flow of 2 kg/min was used, resulting in about 200% increase in volume of the continuous phase of the suspension. The mixing speed was constant at 7000 RPM for all trials.

Finally, the microparticle batches were washed using an ambient water wash with two volume changes for 40 minutes and an air sweep of 80 SLPM, a hot water wash (39°C) with 5 volume changes for 100 minutes with an air sweep of 80 SLPM, and were cooled at 1.5 volume exchange of 30 minutes with an air sweep at 80 SLPM. The air sweep was introduced to aid the head space solvent removal during the wash process. Microparticles were filtered on a filter membrane and freeze dried under a vacuum.

**Table 5**

| Batch | GC071304 | GC06404 | GC060304 | GC061104 |
|---|---|---|---|---|
| Dilution composition, % of continuous phase volume | 0 | 50 | 100 | 200 |
| L.A._{DP} conc., g/g | 0.0483 | 0.0485 | 0.0484 | 0.0485 |
| L.A. _{DP} wt., g | 1.585 | 1.593 | 1.593 | 1.597 |
| L.A.-free_{DP} conc., g/g | 0.0457 | 0.0459 | 0.0458 | 0.0459 |
| R202H_{DP} conc., g/g | 0.170 | 0.170 | 0.170 | 0.170 |
| R202H_{DP} wt., g | 7.06 | 7.09 | 7.05 | 7.05 |
| DCM_{DP} conc., g/g | 0.245 | 0.246 | 0.244 | 0.244 |
| DCM_{DP} wt., g | 15.01 | 15.0 | 15.07 | 15.10 |
| MeOH_{DP} conc., g/g | 0.521 | 0.520 | 0.522 | 0.523 |
| MeOH_{DP} wt., g | 4.904 | 4.899 | 4.904 | 4.909 |
| A. Acid_{DP} conc., g/g | 0.0086 | 0.0084 | 0.0086 | 0.0083 |
| A. Acid_{DP} wt., g | 0.248 | 0.242 | 0.249 | |
| CP Flow Rate, g/min | 2000 | 2000 | 2000 | 2000 |
| DP Flow rate, g/min | 35.41 | 36.88 | 37.45 | 34.55 |
| Mixer, rpm | 7000 | 7000 | 7000 | 7000 |

The properties of the microparticle prepared in Table 5 are shown in Table 6 below.

**Table 6**

| Batch | GC071304 | GC06404 | GC060304 | GC061104 |
|---|---|---|---|---|
| Dilution composition, % of continuous phase volume | 0 | 50 | 100 | 200 |
| Drug Load, % | 14.04±0.11 | 14.17±0.35 | 13.92±0.10 | 14.25±0.12 |
| Part. Size Dist., | 3.58 | 3.44 | 3.51 | 4.55 |
| 10% CVF Under, micron | | | | |
| Part. Size Dist., | 12.3 | 11.6 | 10.9 | 14.2 |
| 25% CVF Under, micron | | | | |
| Part. Size Dist., | 27.0 | 26.4 | 24.6 | 29.2 |
| 50% CVF Under, micron | | | | |
| Part. Size Dist., | 38.8 | 38.2 | 37.2 | 40.7 |
| 75% CVF Under, micron | | | | |
| Part. Size Dist., | 47.6 | 46.8 | 48.8 | 49.9 |
| 90% CVF Under, micron | | | | |
| D. R. (Phys.), 0.2 Day | 0.66±0.01 | | 1.41±0.03 | 1.69±0.03 |
| D. R. (Phys.), 1 Days | 1.09±0.02 | | 1.95±0.04 | 2.80±0.04 |
| D. R. (Phys.), 3 Days | 1.48±0.03 | | 2.30±0.04 | 3.48±0.02 |
| D. R. (Phys.), 7 Days | 3.41±0.12 | | 2.98±0.05 | 5.62±0.04 |
| D. R. (Phys.), 10 Hour | 4.80±0.12 | | 3.63±0.04 | 7.02±0.11 |
| D. R. (Phys.), 14 Hours | 7.79±0.17 | | 6.12±0.15 | 11.66±0.06 |
| D. R. (Phys.), 17 Hours | 9.294±0.16 | | 7.80±0.17 | 13.43±0.04 |
| D. R. (Phys.), 21 Hours | 12.64±0.13 | | 10.53±0.23 | 16.93±0.04 |
| D. R. (Phys.), 24 Hours | 18.02±0.26 | | 15.29±0.33 | 21.40±0.19 |
| D. R. (Phys.), 28 Hours | 24.12±0.26 | | 23.97±0.94 | 28.56±0.47 |
| D. R. (Acc.), 1 Hours | 22.08±1.94 | | 23.10±1.26 | 27.57±0.78 |
| D. R. (Acc.), 5 Hours | 41.56±0.51 | | 40.89±0.36 | 43.28±0.78 |
| D. R. (Acc.), 24 Hours | 61.56±0.04 | | 61.65±0.42 | 62.11±1.30 |
| D. R. (Acc.), 48 Hours | 74.96±1.07 | | 74.60±0.61 | 74.86±1.60 |
| D. R. (Acc.), 72 Hours | 83.82±1.24 | | 83.36±1.11 | 83.51±1.75 |
| D. R. (Acc.), 96 Hours | 89.83±1.23 | | 89.56±1.27 | 89.61±2.09 |

The drug release properties were tested under physiological and accelerated conditions, as described above with regard to Example 1. Drug encapsulation and particle size properties of the microparticles produced from the different compositions were comparable. Encapsulation efficiency of the leuprolide free base ranged from 80 to 82%. Results shows that the drug encapsulation is comparable for all the batches with dilution compositions ranging from 0 to 200% increased volume additions to the continuous phase portion. The average particle size (50% volume distribution under) ranged from 25 to 29 µm and 90% of the volume distribution ranged from 47 to 50 µm. In general, drug encapsulation and particle size distribution are similar among the batches.

The drug release rate measured at physiological condition was measured as an indicator of the real time drug release rate (long-term). The results showed faster release rates as the dilution compositions were increased, and the continuous phase further diluted. The accelerated release rate test conditions also showed slightly higher initial release rates as the dilution compositions in the continuous phase increased. For example, batch GC061104, with the highest amount of dilution composition and the lowest concentration of solvent in the continuous phase portion, showed the fastest release under both release conditions at all time intervals. Batch GC071304 did not have an increase in dilution composition. As the sample with the highest solvent concentration, this sample had the slowest release rates under physiological conditions for time intervals 3 days and under. Therefore, the decrease in solvent exposure after the microparticles are formed increases the release rate of leuprolide from the microparticles.

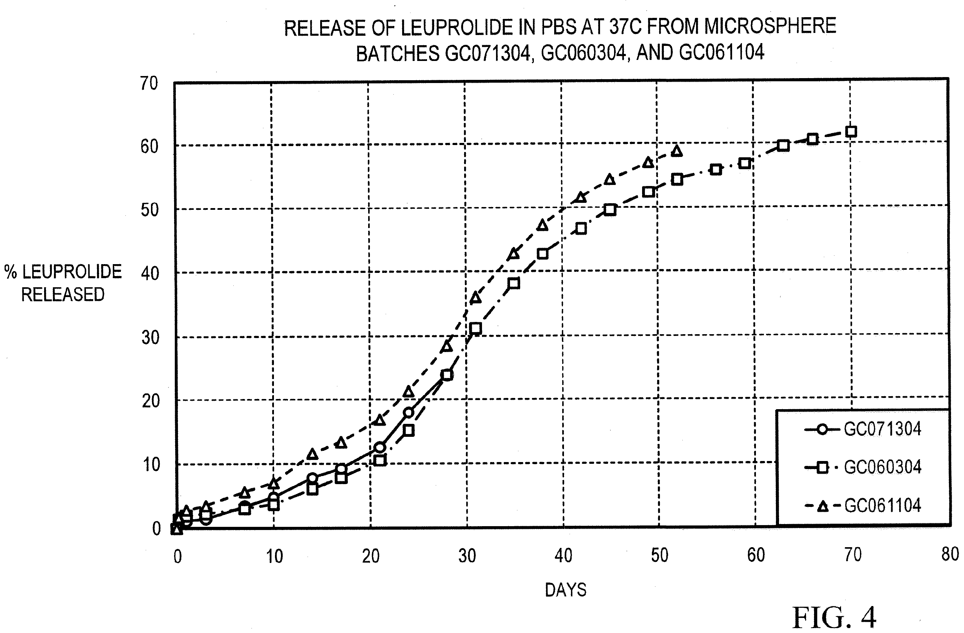

Fig. 4 is a graphic representation of the release properties of leuprolide microparticles in a phosphate buffer solution at 37°C, including batches GC071304, GC060304, and GC061104 as prepared and described in Tables 5 and 6. Fig. 4 displays three S-Shaped curves representing the percent of leuprolide released at 37°C in PBS when the batches are made with different levels of dilution composition. Although there is a narrow separation between all three curves, Fig. 4 shows microparticle batch GC061104, corresponding to the highest dilution composition addition of 200%, had the highest leuprolide release rate, particularly at early time points.

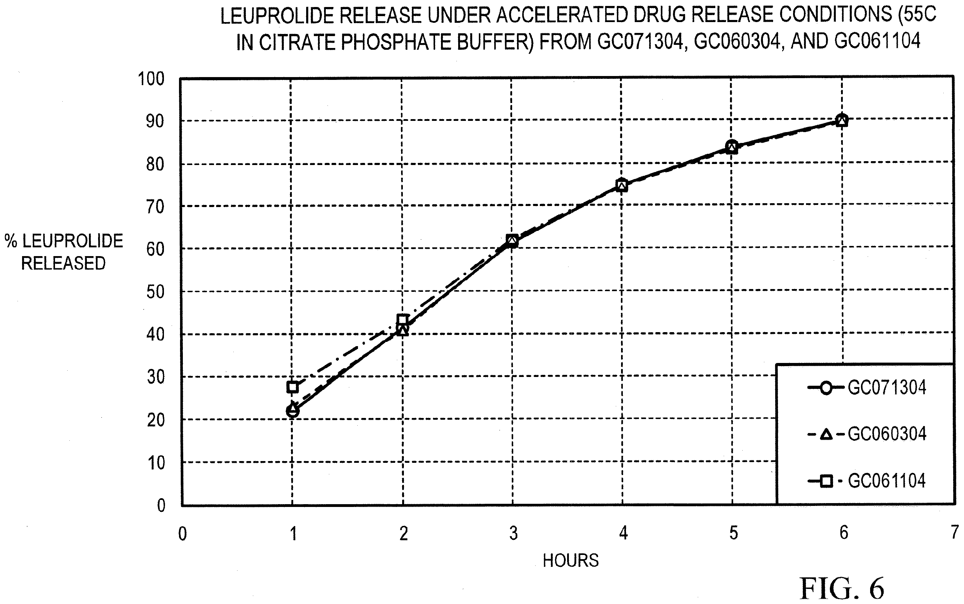

Fig. 6 is a graphic representation of the release properties of leuprolide acetate microparticles under accelerated release conditions in a citrate phosphate buffer solution at 55°C, including batches GC071304, GC060304, and GC061104, as prepared and described in Tables 5 and 6. The microparticle batches were plotted to show the leuprolide release rate according to accelerated release test conditions and recorded against hourly time intervals. For the first data points (1 hour and 5 hour), microparticle batch GC061104 was shown to have a slightly higher release.

### Example 4

Microparticle batches were prepared using leuprolide and R202H with an intrinsic viscosity of 0.157 dL/g and a molecular weight of 11.5 kDa. The batches were made at approximately a 10 g scale.

Table 7 compares the preparation parameters of the batches. Preparation parameters are similar for all the batches except the amount of the dilution composition added.

**Table 7**

| Batch | GC071404 | GC071504 | GC090304 |
|---|---|---|---|
| Dilution composition, % of continuous phase volume | 0 | 100 | 200 |
| L.A._{DP} conc., g/g | 0.0485 | 0.0483 | 0.0482 |
| L.A._{DP} wt., g | 1.5923 | 1.5886 | 1.583 |
| L.A.-free_{DP} conc., g/g | 0.0459 | 0.0457 | 0.456 |
| L.A. -free_{DP} wt., g | | | |
| R202H_{DP} conc., g/g | 0.2451 | 0.2442 | 0.2454 |
| R202H_{DP} wt., g | 7.06 | 7.05 | 7.07 |
| DCM_{DP} conc., g/g | 0.5211 | 0.5224 | 0.5210 |
| DCM_{DP} wt., g | 15.01 | 15.08 | 15.01 |
| MeOH_{DP} conc., g/g | 0.1700 | 0.1698 | 0.1701 |
| MeOH_{DP} wt., g | 4.8967 | 4.9013 | 4.8994 |
| A. Acid_{DP} conc., g/g | 0.0086 | 0.0085 | 0.0085 |
| A. Acid_{DP} wt., g | 0.2471 | 0.2463 | 0.2455 |
| Wt. Dispersed Phase, g | 28.8061 | 28.8362 | 28.8079 |
| Solvent Exposure Duration, post formation, min | 30 | 30 | 30 |
| CP Flow Rate, g/min | 2000 | 2000 | 2000 |
| DP Flow rate, g/min | 35.84 | 36.1 | 34.41 |
| Mixer, rpm | 7000 | 7000 | 7000 |

Table 8 compares the properties of the microparticle batches prepared as in Table 7 with varying amounts of dilution compositions using the 0.157 IV polymer.

**Table 8**

| Batch | GC071404 | GC071504 | GC090304 |
|---|---|---|---|
| Dilution composition, % of continuous phase volume | 0 | 100 | 200 |
| Drug Load, % | 11.85±0.41 | 13.49±0.13 | 13.26±0.21 |
| Part. Size Dist., 10% CVF Under, micron | 4.42 | 3.37 | 3.49 |
| Part. Size Dist., 25% CVF Under, micron | 14.3 | 11.6 | 15.3 |
| Part. Size Dist., 50% CVF Under, micron | 24.0 | 22.7 | 25.7 |
| Part. Size Dist., 75% CVF Under, micron | 32.4 | 32.5 | 34.6 |
| Part. Size Dist., 90% CVF Under, micron | 39.3 | 40.3 | 42.1 |
| D. R. (Phys.), 0.2 Day | 1.34±0.04 | 16.62±0.54 | 10.83±0.08 |
| D. R. (Phys.), 1 Days | 3.16±0.01 | 15.63±0.11 | 15.84±0.21 |
| D. R. (Phys.), 3 Days | 5.76±0.10 | 25.17±0.06 | 23.49±0.52 |
| D. R. (Phys.), 7 Days | 16.87±0.50 | 35.73±0.15 | 33.65±0.50 |
| D. R. (Phys.), 10 Days | 30.17±0.54 | 41.18±0.29 | 38.90±0.50 |
| D. R. (Phys.), 14 Days | 41.28±0.36 | 45.55±0.34 | 43.20±0.55 |
| D. R. (Phys.), 17 Days | 49.14±0.55 | 48.60±0.67 | 46.51±0.63 |
| D. R. (Phys.), 21 Days | 55.80±0.74 | 51.82±0.94 | 50.51±0.82 |
| D. R. (Phys.), 24 Days | 62.12±0.98 | 54.49±1.30 | 53.86±0.98 |
| D. R. (Phys.), 28 Days | 69.75±0.38 | 58.34±1.11 | 58.21±1.25 |
| D. R. (Phys.), 31 Days | | 61.38±1.2 | 61.98±1.11 |
| D. R. (Acc.), 1 Hours | 16.94±0.54 | 28.16±0.72 | 27.31±0.86 |
| D. R. (Acc.), 5 Hours | 44.83±1.54 | 52.84±1.55 | 52.82±1.39 |
| D. R. (Acc.), 24 Hours | 81.23±0.44 | 81.49±3.89 | 83.41±0.45 |
| D. R. (Acc.), 48 Hours | 94.31±0.27 | 93.99±0.18 | 94.55±0.35 |
| D. R. (Acc.), 72 Hours | 98.40±0.22 | 97.53±1.30 | 97.73±0.04 |
| D. R. (Acc.), 96 Hours | 98.03±0.36 | 97.64±1.55 | 97.6±0.16 |

The microparticles prepared without the addition of the dilution composition to the continuous phase had comparatively lower release rates. The lower molecular weight polymer is more sensitive to the solvent exposure and drug content in the microparticle than those microparticles made with the higher molecular weight polymer. The particle size distribution is comparable for the three batches and the variation is small with an average particle size (50% volume distribution under) ranging from 23 to 26 µm. Batch GC071404 produced with no dilution composition showed a slower initial release than did the other two batches. This difference in early release is reflected by the accelerated in-vitro release also.

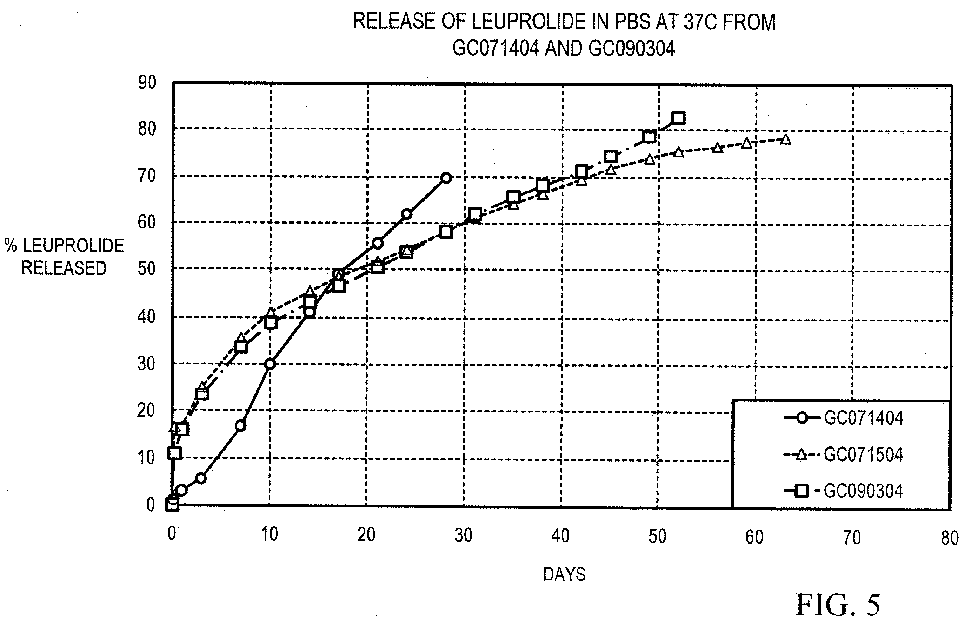

Fig. 5 compares the drug release rates in a phosphate buffer solution for the microparticles formed with the polymer having an intrinsic viscosity of 0.157. The microparticles formed with the 0.157 IV polymer showed much slower early release in the zero dilution composition batch. For the batches with 100 and 200% dilution composition additions, the 0.157 IV polymer microparticles showed higher release rates. The addition of the dilution composition minimizes the solvent exposure level to the freshly formed microparticle and increases the release rate for the low IV polymers.

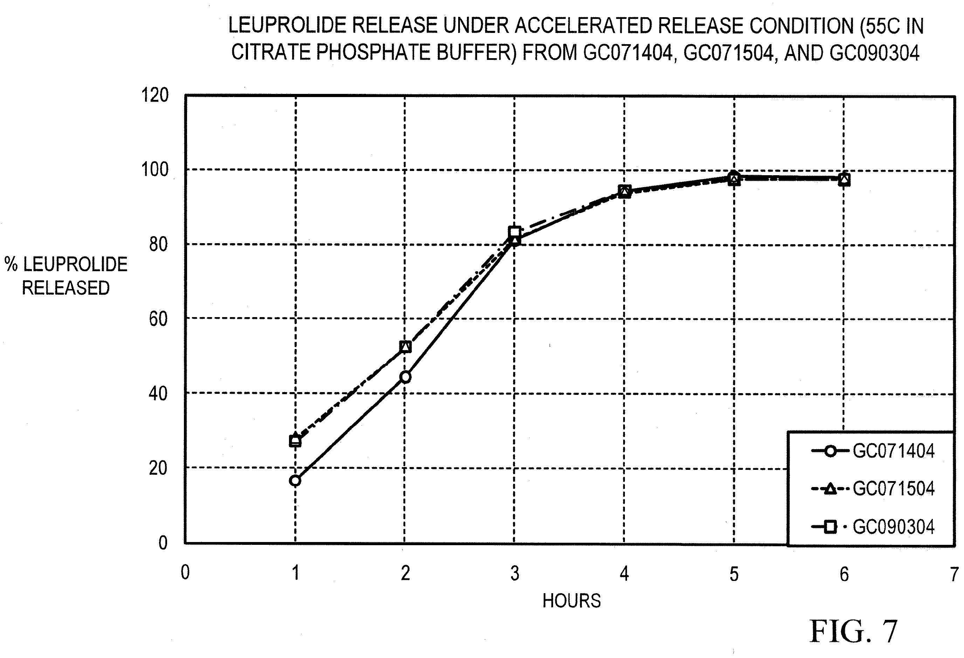

Fig. 7 is a graphic representation of three samples having varying amounts of dilution compositions as set forth in Table 7, with the percentage of leuprolide acetate released plotted against time in hours under accelerated in-vitro conditions. The batches with increased dilution compositions have greater release rates in the first two weeks under physiological conditions and in the first five hours under accelerated release test conditions.

### Example 5

Example 5 details large scale microparticle batches with and without dilution compositions added to the continuous phase. Pharmacokinetics (PK) and efficacy data and for three leuprolide microparticle batches (0E5103, 0B6315 and 0A0307) were prepared using R202H polymer which has an intrinsic viscosity of approximately 0.21 dL/g. Batches 0E5103 and 0A0307 were tested in humans with 22.5 mg doses for pharmacokinetics and efficacy purposes. The pharmacokinetic (PK) groups of patients were monitored for blood (serum) leuprolide level and testosterone suppression and the remaining patients were monitored for efficacy only (testosterone suppression). To show chemical castration using leuprolide, 95% of the patients must show testosterone levels at or below 50 ng/dL (0.5 ng/mL) by 28 days or earlier.

Microparticle batch 0E5103 was prepared with R202H having 0.206 IV and a molecular weight of 18 kDa without any addition of dilution composition or dilution. Methylene chloride exposure for this batch was approximately 7200 ppm and the duration of exposure was approximately 40 minutes. Microparticle batch 0B6315 was prepared with poly(D,L-lactide) having 0.206 IV and a molecular weight of approximately 18 kDa. The dilution composition was prepared by adding 150% of water to the continuous phase flow while the continuous phase is in the solvent removal vessel.

Microparticle batch 0A0307 was prepared using poly(d,l-lactide) having a similar IV and molecular weight to batch 0B6315. The dilution composition was prepared by adding 200% of water to the continuous phase flow while the continuous phase and the dispersed phase emulsion is fed through the in-line mixer into the solvent removal vessel. The methylene chloride solvent level was approximately 1500 ppm in the continuous phase as determined by gas chromatography (GC). Table 9 compares the preparation parameters and Table 10 compares the properties of the microparticles prepared in table 9.

Notably, the batch size for 0A0307 is at approximately 1 Kg scale and the microparticle formation time was 100 minutes in the reactor vessel. Batch 0E5103 has a batch size of 0.4 Kg scale and the microparticle formation time was 40 minutes. The formulation for the dispersed phase was similar for the three batches, with the 0E5103 batch having a slightly increased active agent and polymer concentration and a decreased concentration of a solvent (methanol) and acetic acid.

**Table 9**

| Batch | 0E5103 | 0B6315 | 0A0307 |
|---|---|---|---|
| Dilution composition, % of continuous phase | 0 | 150 | 200 |
| Polymer M_{w}, kDa | 18 | 18 | 19 |
| Batch Size, Kg | 0.4 | 0.4 | 1.0 |
| L.A. _{DP} wt., g | 72 | 77 | 202 |
| L.A.-free_{DP} conc., g/g | 0.048 | 0.0434 | 0.044 |
| R202H_{DP} conc., g/g | 0.25 | 0.21 | 0.21 |
| R202H_{DP} wt., g | 353 | 353 | 820 |
| DCM_{DP} conc., g/g | 0.53 | 0.53 | 0.53 |
| DCM_{DP} wt., g | 750 | 900 | 2100 |
| MeOH_{DP} conc., g/g | 0.18 | 0.19 | 0.19 |
| MeOH_{DP} wt., g | 236 | 324 | 755 |
| A. Acid_{DP} conc., g/g | 0.004 | 0.0165 | 0.0165 |
| A. Acid_{DP} wt., g | 6 | 27 | 65 |
| Solvent Exposure Duration, post formation. | 7200 ppm DCM for up to 40 min | 3000 ppm DCM for up to 40 min | 1500 ppm DCM for up to 100 min |
| CP Flow Rate, L/min | 2 | 2 | 2 |
| DP Flow rate, g/min | 37 | 35 | 35 |
| Mixer, rpm | 7000 | 7000 | 7000 |
| Dilution composition Addition rate, L/min | 0 | 3 | 4 |

**Table 10**

| Batch | 0E5103 | 0B6315 | 0A0307 |
|---|---|---|---|
| Dilution composition, % of continuous phase volume | 0 | 150 | 200 |
| Drug Load, % | 13.9 | 14.6 | 14.7 |
| Part. Size Dist., | 3 | 4 | 3 |
| 10% CVF Under, micron | | | |
| Part. Size Dist., | 11 | 12 | 11 |
| 25% CVF Under, micron | | | |
| Part. Size Dist., | 26 | 24 | 24 |
| 50% CVF Under, micron | | | |
| Part. Size Dist., | 39 | 33 | 35 |
| 75% CVF Under, micron | | | |
| Part. Size Dist., | 50 | 40 | 46 |
| 90% CVF Under, micron | | | |
| D. R. (Acc.), 1 Hours (%) | 16 | 28 | 37 |
| D. R. (Acc.), 5 Hours (%) | 36 | 46 | 52 |
| D. R. (Acc.), 24 Hours (%) | 59 | 67 | 67 |
| D. R. (Acc.), 48 Hours (%) | 73 | 80 | 77 |
| D. R. (Acc.), 72 Hours (%) | 81 | 87 | 83 |
| Residual DCM in product, ppm | <100 | <100 | <100 |
| Residual MeOH in product, ppm | <100 | <100 | <100 |

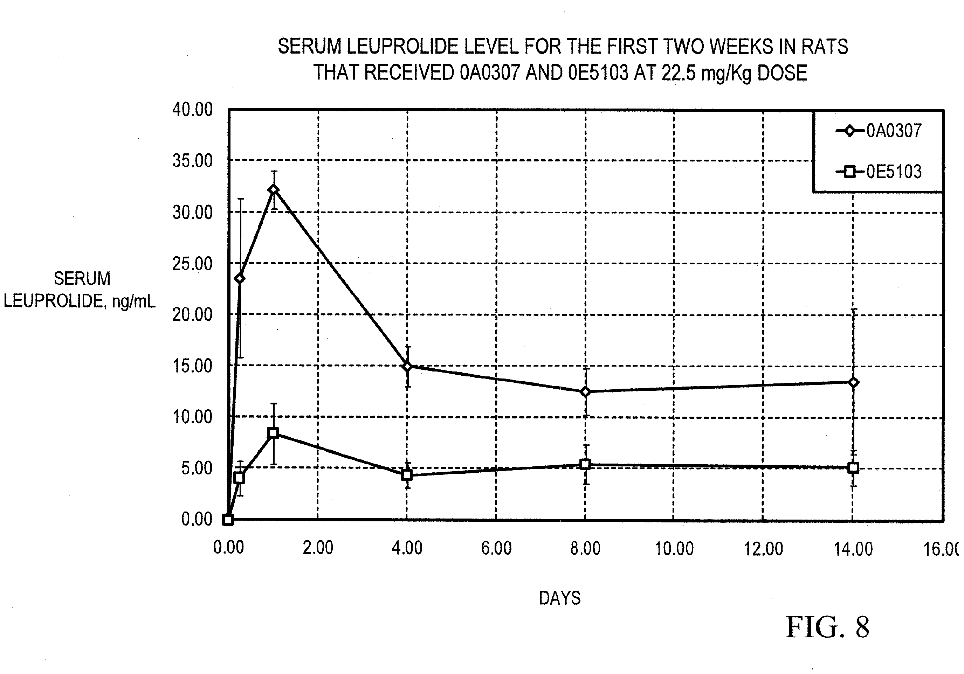

The accelerated in-vitro release rates showed that 0B6315 and 0A0307 with an increased dilution composition added and therefore, a reduced solvent exposure, produced microparticles with higher initial release compared to 0E5103 that does not have the dilution composition added. Fig. 8 compares the serum leuprolide concentrations in rats given a dose of 22.5 mg leuprolide per Kg for batches 0E5103 and 0A0307 for a two weeks period. Batch 0A03070A0307, with the lowest solvent exposure, had a higher early release compared to 0E5103, producing higher concentrations of leuprolide in serum.

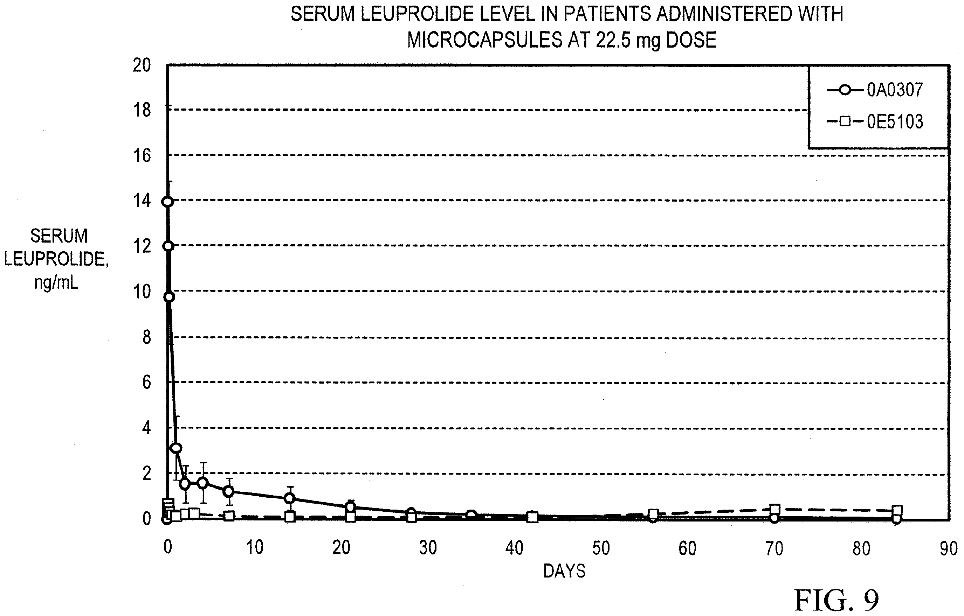

Fig. 9 is a graphic representation of serum leuprolide concentration levels (ng/ml) in humans administered with leuprolide microparticles prepared according to Table 9. Microparticles batches 0E5103 and 0A0307 were prepared and described in Tables 9. Table 10 shows human clinical data of the blood (serum) leuprolide concentration. The results of this example shows that the formulation with the dilution composition (0A0307) and low DCM exposure had higher leuprolide level in blood during the initial one month period due to faster release. This batch worked more effectively as GnRH agonist in human system to achieve a long term reduction in testosterone levels. Patients who received 0A0307 at the same dose had lower leuprolide level in blood and the drug was released at a faster rate during the later part (after one month) of the study.

A clinical trial comparing batches 0E5103 and 0A0307 was performed to compare the testosterone levels in patients after receiving leuprolide microparticles with varying levels of dilution compositions added after the microparticle formation. Ideally, 95% of the patients should achieve and maintain a testosterone level below 50 ng/dL in 28 days. Table 11 details the clinical trial results of the two leuprolide microparticle batches.

**Table 11**

| Data Set | % Patients achieving testosterone level of less than 50 ng/dl after 28 days |
|---|---|
| Clinical Requirement | 95 |
| 0E5103 | 79 |
| 0A0307 | 98 |

Due to slow initial release from batch 0E5103, only 79% of the patients achieved the desired level by day 28. However, patients who received batch 0A0307, the batch with the 200% dilution composition, achieved the desired level in 98% of the population by day 28. As a result, patients with discomfort and pain due to higher testosterone levels in illnesses such as prostate cancer can be treated more effectively and have their pain reduced more quickly by administering a sustained release drug with an initial higher release rate of leuprolide.

### Example 6

The batches for example 6 were prepared at approximately 10 g scale using a R202H with an inherent viscosity of 0.183 IV. Table 12 compares the preparation parameters for the batches. The dispersed phase composition and the continuous phase composition were same for all the batches and the target load was 16.5% L.A for all the batches. The flow rate for the continuous phase and the dispersed phase were the same or comparable.

**Table 12**

| Batch | GC061404 | GC061504 | GC061604 | GC061704 |
|---|---|---|---|---|
| Dilution Composition, % of Continuous Phase Volume | 0 | 50 | 100 | 200 |
| L.A._{DP} conc., g/g | 0.0484 | 0.0483 | 0.0485 | 0.0483 |
| L.A_{DP} wt., g | 1.586 | 1.588 | 1.594 | 1.587 |
| L.A.-free_{DP}. conc., g/g | 1.3165 | 1.3180 | 1.3228 | 1.3175 |
| L-free_{DP}, wt., g | 0.0457 | 0.0457 | 0.0459 | 0.0457 |
| R202H_{DP} conc., g/g | 7.06 | 7.11 | 7.09 | 7.07 |
| R202H_{DP} wt., g | 0.245 | 0.246 | 0.246 | 0.245 |
| DCM_{DP} conc., g/g | 15.0 | 15.0 | 15.01 | 15.00 |
| DCM_{DP} wt., g | 0.521 | 0.520 | 0.521 | 0.521 |
| MeOH_{DP} conc., g/g | 4.895 | 4.909 | 4.906 | 4.907 |
| MeOH_{DP} wt., g | 0.170 | 0.170 | 0.170 | 0.170 |
| PVA conc., in CP, g/g | 0.0035 | 0.0035 | 0.0035 | 0.0035 |
| CP Flow rate, g/min | 2000 | 2000 | 2000 | 2000 |
| DP Flow rate, g/min | 34.25 | 34.25 | 34.27 | 34.29 |
| Silverson rpm | 7000 | 7000 | 7000 | 7000 |

Table 13 compares the properties of the microparticle batches.

**Table 13**

| Batch | GC061404 | GC061504 | GC061604 | GC061704 |
|---|---|---|---|---|
| Dilution composition, % of continuous phase volume | 0 | 50 | 100 | 200 |
| Drug load, % | 13.25±0.17 | 13.85±0.09 | 13.91±0.17 | 13.51±0.07 |
| Part. Size Dist., | 2.88 | 2.99 | 3.52 | 3.94 |
| 10% CVF Under, micron | | | | |
| Part. Size Dist., | 9.73 | 9.13 | 11.7 | 12.4 |
| 25% CVF Under, micron | | | | |
| Part. Size Dist., | 22.5 | 22.4 | 27.8 | 24.9 |
| 50% CVF Under, micron | | | | |
| Part. Size Dist., | 33.6 | 32.6 | 38.0 | 35.1 |
| 75% CVF Under, micron | | | | |
| Part. Size Dist., | 41.9 | 40.1 | 45.8 | 42.9 |
| 90% CVF Under, micron | | | | |
| D. R. (Phys.), 0.2 Day | 0.44±0.0 | 0.95±0.07 | 1.03±0.02 | 1.14±0.02 |
| D. R. (Phys.), 1 Day | 0.57±0.01 | 1.19±0.06 | 1.36±0.03 | 1.39±0.04 |
| D. R. (Phys.), 3 Day | 0.95±0.03 | 2.11±0.03 | 2.34±0.09 | 2.34±0.06 |
| D. R. (Phys.), 7 Day | 5.65±0.28 | 6.64±0.37 | 5.94±0.18 | 5.39±0.20 |
| D. R. (Phys.), 10 Day | 8.45±0.34 | 11.48±0.25 | 10.62±0.73 | 9.87±0.59 |
| D. R. (Phys.), 14 Day | 14.28±0.58 | 21.54±0.61 | 19.86±0.61 | 17.69±1.62 |
| D. R. (Phys.), 17 Day | 21.77±0.92 | 28.99±0.87 | 27.61±0.61 | 24.37±2.15 |
| D. R. (Phys.), 21 Day | 30.21±0.73 | 34.94±0.73 | 34.43±0.36 | 31.72±1.83 |
| D. R. (Phys.), 24 Day | 35.93±0.29 | 39.00±0.60 | 39.00±0.22 | 37.06±1.23 |
| D. R. (Phys.), 28Day | 40.59±0.23 | 42.82±0.54 | 43.15±0.29 | 41.53±0.99 |
| D. R. (Phys.), 31 Day | 44.20±0.30 | 45.90±0.67 | 46.30±0.43 | 44.67+0.87 |
| D. R. (Acc.), 1 Hr Release | 15.32±0.11 | 21.26±0.22 | 23.75±0.51 | 21.14±0.66 |
| D. R. (Acc.), 5 Hr Release | 38.35±0.21 | 42.80±0.40 | 44.71±0.42 | 43.06±0.34 |
| D. R. (Acc.), 24 Hr Release | 68.75±0.32 | 70.95±0.26 | 71.84±0.67 | 71.75±0.27 |
| D. R. (Acc.), 48 Hr Release | 83.14±0.78 | 84.68±0.22 | 84.68±0.31 | 85.12±1.08 |
| D. R. (Acc.), 72 Hr. Release | 91.30±0.29 | 91.93±0.25 | 91.55±0.30 | 92.94±0.83 |
| D. R. (Acc.), 96 Hr. Release | 95.70±0.88 | 95.96±0.60 | 94.99±0.35 | 97.18±0.75 |

Drug load in the microparticles was comparable for the batches. The particle size showed small, typical, variation among the batches. The average particle size (50% volume distribution under) ranged from 22 to 28 µm, and 90% of the volume distribution ranged from 40 to 46 µm. In general, particle size did not show much difference among batches, and there is no defined trend.

The drug release at 37°C in PBS is expected to release the drug for more than three months. The initial release for a minimum one-month period was followed in PBS. The batch with no dilution composition (GC061404) showed a lower initial release compared to the batches prepared with dilution composition. The batches with 50, 100 and 200% dilution composition showed faster, comparable release rates.

The accelerated release method also showed a slightly higher initial release (1 and 5 hour) for the batches prepared with a dilution composition. There is no difference in release among the batches prepared with varying dilutions. The increase in early release (1 and 5 hour) by dilution was achieved with 50% dilution. Further dilution did not improve the initial release at a notable magnitude.

### Example 7

Three microparticle batches were prepared with 100, 200 and 300 % dilution composition using R202H with an inherent viscosity of 0.206 IV. The preparation parameters are shown in Table 14 for GC071204, GC090904, and GC091004. The preparation parameters were comparable for these batches except the intentional variable, dilution composition.

**Table 14**

| Batch | GC071204 | GC090904 | GC091004 |
|---|---|---|---|
| Dilution Composition, % of continuous phase volume | 100 | 200 | 300 |
| LA _{DP} conc., g/g | 0.0488 | 0.0477 | 0.0487 |
| LA._{DP} wt., g | 1.6614 | 1.6475 | 1.6662 |
| L-free _{DP} wt., g | 1.5717 | 1.5585 | 1.5762 |
| R202H _{DP} conc., g/g | 0.2084 | 0.2040 | 0.2082 |
| R202H _{DP} wt., g | 7.09 | 7.05 | 7.12 |
| DCM _{DP} conc., g/g | 0.5293 | 0.5382 | 0.5301 |
| DCM_{DP} wt., g | 18.01 | 18.6 | 18.13 |
| Methanol _{DP} conc., g/g | 0.1906 | 0.1873 | 0.1902 |
| MeOH_{DP} wt., g | 6.4851 | 6.4741 | 6.5035 |
| A. Acid _{DP} conc., g/g | 0.0160 | 0.0162 | 0.0160 |
| A. Acid _{DP} wt., g | 0.5454 | 0.5585 | 0.5475 |
| PVA _{CP} conc., g/g | 0.0035 | 0.0035 | 0.0035 |
| CP Flow rate, g/min | 2000 | 2000 | 2000 |
| DP Flow rate, g/min | 33.90 | 35.14 | 35.18 |
| Silverson RPM | 7000 | 7000 | 7000 |

The properties of the batches prepared in Table 14 are shown below in Table 15. The final microparticle had 71 to 75% encapsulation efficiency and the drug load was comparable for the batches. The batches with 100 and 200% dilution showed comparable particle size. Slightly faster release rates (or comparable release rates) were observed in PBS during the earlier time points for the batches prepared with higher dilutions. Accelerated release showed comparable early release.

**Table 15**

| Batch | GC071204 | GC090904 | GC091004 |
|---|---|---|---|
| Dilution Composition, % of continuous phase | 100 | 200 | 300 |
| LA Load in final MS, % | 15.04±0.34 | 14.91±0.01 | 14.21±0.15 |
| Part. Size Dist., | 3.91 | 4.19 | 5.88 |
| 10% CVF Under, micron | | | |
| Part. Size Dist., | 11.4 | 12.0 | 18.7 |
| 25% CVF Under, micron | | | |
| Part. Size Dist., | 23.2 | 23.9 | 29.3 |
| 50% CVF Under, micron | | | |
| Part. Size Dist., | 33.0 | 33.2 | 38.0 |
| 75% CVF Under, micron | | | |
| Part. Size Dist., | 41.7 | 41.1 | 46.2 |
| 90% CVF Under, micron | | | |
| D.R. (Phys.), 0.2 Day | 5.28±0.08 | 8.53±0.26 | 10.12±0.34 |
| D.R. (Phys.), 1 Day | 12.41±0.25 | 17.13±0.99 | 19.46±0.60 |
| D.R. (Phys.), 3 Day | 14.80±1.40 | 20.35±1.62 | 24.85±1.04 |
| D.R. (Phys.), 7 Day | 23.62±1.83 | 25.69±2.64 | 28.90±1.05 |
| D.R. (Phys.), 10 Day | 28.16±1.73 | 29.56±3.41 | 31.93±1.02 |
| D.R. (Phys.), 14 Day | 33.03±1.51 | 33.59±2.79 | 35.06±1.05 |
| D.R. (Phys.), 17 Day | 38.67±1.41 | 36.99±2.41 | 37.37±1.03 |
| D.R. (Phys.), 21 Day | 42.73±1.24 | 41.09±2.08 | 40.13±1.09 |
| D.R. (Phys.), 24 Day | 46.24±1.24 | 44.45±1.75 | 42.52±1.18 |
| D.R. (Phys.), 28 Day | 49.84±1.05 | 47.82±1.34 | 44.88±1.30 |
| D.R. (Acc.), 1 Hr Release | 33.56±0.96 | 32.55±0.62 | 30.24±0.09 |
| D.R. (Acc.), 5 Hr Release | 49.53±0.17 | 48.84±0.47 | 46.20±0.38 |
| D.R. (Acc.), 24 Hr Release | 67.35±0.28 | 68.65±0.64 | 66.84±0.54 |
| D.R. (Acc.), 48 Hr Release | 78.52±0.33 | 78.39±0.53 | 77.21±0.41 |
| D.R. (Acc.), 72 Hr. Release | 85.58±0.52 | 86.37±0.34 | 85.44±0.61 |
| D.R. (Acc.), 96 Hr. Release | 91.26±0.31 | 92.21±0.30 | 91.35±0.81 |

### Example 8

Three microparticle batches were prepared with 100, 200 and 300 % dilution composition, using R202H with in inherent viscosity of 0.157 IV and a 19% L.A. target load. The preparation parameters are shown in Table 16. The preparation parameters were comparable for the batches except the intentional variable, dilution composition.

**Table 16**

| Batch | GC071604 | GC090204 | GC090704 |
|---|---|---|---|
| Dilution, Composition, % | 100 | 200 | 300 |
| LA _{DP} conc., g/g | 0.0488 | 0.0488 | 0.0488 |
| LA _{DP} wt., g | 1.6613 | 1.6590 | 1.6587 |
| L-FB _{DP} wt., g | 1.5715 | 1.5694 | 1.5691 |
| R202H _{DP} conc., g/g | 7.08 | 7.10 | 7.05 |
| R202H _{DP} wt., g | 0.208 | 0.2086 | 0.2074 |
| DCM _{DP} conc., g/g | 18.04 | 18.0 | 18.0 |
| DCM _{DP} wt., g | 0.5299 | 0.5289 | 0.5296 |
| Methanol _{DP} conc., g/g | 6.4753 | 6.4920 | 6.487 |
| MeOH _{DP} wt., g | 0.1902 | 0.1908 | 0.1908 |
| A. Acid _{DP} conc., g/g | 0.5542 | 0.5475 | 0.5628 |
| A. Acid _{DP}, wt., g | 0.0163 | 0.0161 | 0.0166 |
| PVA _{CP} wt.,, g/g | 0.0035 | 0.0035 | 0.0035 |
| Silverson RPM | 7000 | 7000 | 7000 |

Table 17 shows the properties of the microparticle batches prepared above. The final batches had microparticles with 69 to 73% encapsulation efficiency. The drug load was comparable for the batches, at around 14%. The particle size is comparable for the batches. The drug release rates were comparable for all the batches in PBS and accelerated release showed slightly higher initial release.

**Table 17**

| Batch | GC071604 | GC090204 | GC090704 |
|---|---|---|---|
| Dilution Composition, % | 100 | 200 | 300 |
| LA Load, % | 13.97±0.00 | 13.99±0.05 | 13.90±0.17 |
| Part. Size Dist., 10% CVF under, micron | 3.91 | 3.63 | 4.91 |
| Part. Size Dist., 25% CVF under, micron | 11.7 | 8.59 | 13.8 |
| Part. Size Dist., 50% CVF under, micron | 21.1 | 19.4 | 23.6 |
| Part. Size Dist., 75% CVF under, micron | 28.2 | 28.3 | 31.4 |
| Part. Size Dist., 90% CVF under, micron | 34.7 | 35.3 | 38.3 |
| D.R. (Phys.), 0.2 Day | 22.12±1.23 | 16.93±0.08 | 20.66±1.22 |
| D.R. (Phys.), 1 Day | 19.47±0.21 | 22.28±0.08 | 24.74±1.19 |
| D.R. (Phys.), 3 Day | 28.74±0.25 | 28.50±0.50 | 28.45±3.00 |
| D.R. (Phys.), 7 Day | 39.89±0.13 | 38.33±0.41 | 39.14±2.55 |
| D.R. (Phys.), 10 Day | 45.61±0.07 | 43.86±0.51 | 44.83±2.54 |
| D.R. (Phys.), 14 Day | 50.15±0.14 | 48.80±0.58 | 49.72±2.52 |
| D.R. (Phys.), 17 Day | 53.34±0.27 | 52.59±0.65 | 53.35±2.52 |
| D.R. (Phys.), 21 Day | 56.58±0.46 | 56.78±0.85 | 57.34±2.50 |
| D.R. (Phys.), 24 Day | 59.30±0.61 | 60.11±0.94 | 60.56±2.49 |
| D.R. (Phys.), 28 Day | 62.84±0.51 | 63.89±1.04 | 64.42±2.58 |
| D.R. (Acc.), 1 Hr | 32.64±1.44 | 33.46±0.43 | 36.55±0.24 |
| D.R. (Acc.), 5 Hr | 56.64±2.69 | 58.98±1.12 | 61.58±0.13 |
| D.R. (Acc.), 24 Hr | 83.60±4.54 | 86.31±0.34 | 88.01±0.48 |
| D.R. (Acc.), 48 Hr | 93.67±2.10 | 97.08±0.44 | 97.12±0.38 |
| D.R. (Acc.), 72 Hr. | 95.55±1.66 | 98.61±0.88 | 98.41±0.32 |
| D.R. (Acc.), 96 Hr. | 95.42±1.54 | 98.66±1.05 | 98.02±0.19 |

### Example 9

Example 9 compares octreotide microparticles with varying solvent exposure. Octreotide containing poly(d,l-lactide -co-glycolide) microparticles were prepared with similar techniques to the preparation procedure of leuprolide microparticle. A dispersed phase containing PLGA (85:15 PLGA from Alkermes), octreotide, methylene chloride, methanol, and glacial acetic acid was delivered through the dispersed phase inlet tube of the modified Silverson in-line mixer, as explained in US Pat. 5,945,126, at approximately 18 mL per minute. The target load for the octreotide in dispersion was 10%. A continuous phase of 0.35% polyvinyl alcohol in water was delivered at 2 L/min through the continuous phase inlet port of the in-line Silverson. The Silverson mixing speed was 5500 RPM. Detailed preparation parameters can be seen in Table 18, below. Approximately 2L of the suspension produced was then exposed to 15L of the continuous phase, while being constantly stirred. This 15L continuous phase included The continuous phase included 2000, 4000, or 6000 ppm, respectively of methylene chloride. Freshly formed microparticles were exposed for 100 minutes before washing. The control batch did not go through solvent exposure and underwent direct washing.

**Table 18**

| Batch | GJ030804 | GJ030204 | GJ030404 | GJ030904 |
|---|---|---|---|---|
| Octreotide_{DP} wt., g | 0.72 | 0.72 | 0.72 | 0.72 |
| PLGA_{DP} wt., g | 6.48 | 6.48 | 6.48 | 6.48 |
| DCM_{DP} wt., g | 10.14 | 10.14 | 10.14 | 10.14 |
| MeOH_{DP} wt., g | 1.02 | 1.02 | 1.02 | 1.02 |
| A. Acid_{DP} wt., g | 0.78 | 0.78 | 0.78 | 0.78 |
| DCM_{CP} conc., ppm | 0 | 2000 | 4000 | 6000 |
| MeOH_{CP} conc., ppm | 0 | 200 | 400 | 600 |
| A. Acid_{CP} conc., ppm | 0 | 150 | 300 | 450 |

An accelerated in-vivo test was performed, comparing the respective batches of microparticles. The tests were conducted in an acetate buffer solution with a pH of 4.0 at 60°C. The release rates were also calculated for the batches under physiological conditions at 37°C for one day.

**Table 19**

| Batch | GJ030804 | GJ030204 | GJ030404 | GJ030904 |
|---|---|---|---|---|
| Drug Load, % composition in continuous phase | 7.7 | 7.0 | 7.6 | 7.5 |
| Encapsulation Efficiency, % | 77 | 70 | 76 | 75 |
| Part. Size Dist., 10% CVF Under, micron | 7.13 | 4.74 | 3.91 | 7.73 |
| Part. Size Dist., 25% CVF Under, micron | 13.5 | 17.5 | 12.5 | 18.8 |
| Part. Size Dist., 50% CVF Under, micron | 28.0 | 30.5 | 26.7 | 32.7 |
| Part. Size Dist., 75% CVF Under, micron | 38.6 | 42.0 | 39.0 | 46.3 |
| Part. Size Dist., 90% CVF Under, micron | 47.4 | 51.8 | 48.8 | 56.6 |
| D. R. (Acc.), 1.5 Hours | 35.6±1.4 | 41.5±1.1 | 42.0±1.1 | 47.2±7.0 |
| D. R. (Acc.), 3 Hours | 42.9±2.8 | 48.1±0.8 | 48.5±1.4 | 55.4±4.1 |
| D. R. (Acc.), 18 Hours | 62.9±2.5 | 68.1±1.1 | 66.9±0.6 | 69.8±3.5 |
| D. R. (Acc.), 24 Hours | 71.0±2.9 | 76.8±2.8 | 77.1±1.3 | 75.6±1.5 |
| D. R. (Acc.), 48 Hours | 83.6±2.5 | 89.6±0.9 | 85.5±1.8 | 76.7±0.2 |
| D. R. (Acc.), 66 Hours | 97.9±0.2 | 99.0±0.4 | 97.1±0.8 | 97.5±0.3 |
| D. R. (Phys.), 1 Day | 9.5 | 11.6 | 12.4 | 14.6 |

As shown in Table 19, the microparticles exposed to higher amounts of solvent showed higher initial release in the release medium at early time points. Higher initial burst could pose a safety concern as the insulin-like growth factor-1 (IGF-1) could decrease too rapidly in response to the faster release rate. The lower initial release rate for octreotide in a sustained microparticle release formulation would be optimal. Unlike leuprolide, by using a formulation with reduced solvent exposure for the octreotide microparticles, the release rate is reduced.

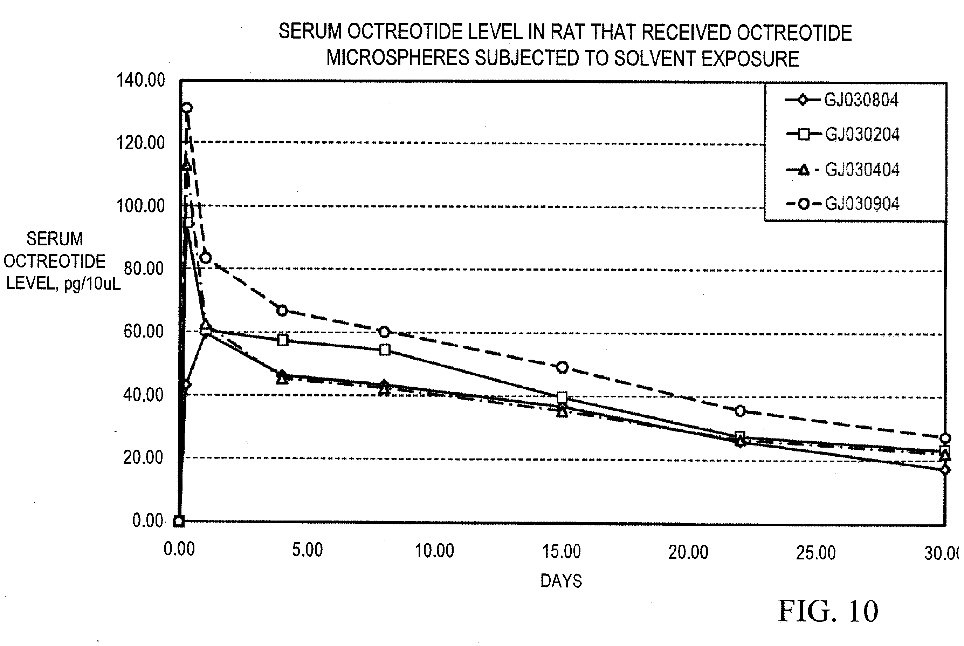

Fig. 10 is a graphic representation of the release properties of the microparticles prepared in accordance to Table 18 in an in-vivo study of octreotide in rats. The rats were given a dose of 5 mg of the octreotide microparticle batches. GJ030804 was not exposed to increased solvent in the continuous phase and showed a lower initial burst release, as compared to the batches prepared with higher concentrations of methylene chloride. Because the higher solvent exposure caused higher burst release rates, it is surmised that microparticles with an even lower burst could be prepared by water dilution, depending on the preferred rate.

### Example 10

Example 10 compares the effect on the drug release rate of leuprolide in microparticles when the batch sizes of the microparticles are varied with and without adding a dilution composition. First, batches GC071304, 0E5103, and 0D4022 were prepared with varied batch size without adding a dilution composition to the continuous phase. As shown in Table 20 below, an 8g batch, GC071304, was prepared using 29 g of dispersed phase, including R202H with a molecular weight of 18 kDa, leuprolide acetate, methylene chloride, and methanol. The continuous phase of GC071304 included 1.5 L of 0.35% polyvinyl alcohol in water. Microparticle formation was completed within 50 seconds at a flow rate of 30-35 g/min for the dispersed phase and 2L/min for the continuous phase. The batch was stirred constantly at 7000 rpm in an in-line line mixer and then washed in ambient and 39°C water with approximately 10 volume changes.

Batch 0E5103 was prepared using the same preparation parameters as above, but the size of the batch was increased to 400 g. The microparticle formation time was 40 minutes. Increasing the batch size to 700 g, batch 0D4022 was prepared using the same preparation parameters as above. The microparticle formation time was 70 min. The amounts of the dispersed phase and continuous phase used in the latter two batches were adjusted proportionately to produce the batch size being considered.

As shown in Table 20 below, three batches were prepared using a 0.206 IV polymer. Table 21 compares the properties of the batches.

**Table 20**

| Batch | GC071304 | 0E5103 | 0D4022 |
|---|---|---|---|
| Batch Size, g | 8 | 400 | 700 |
| Polymer Mw, kDa | 18 | 18 | 18 |
| L.A._{DP}conc., g/g | 0.05 | 0.05 | 0.05 |
| R202H_{DP} conc., g/g | 0.25 | 0.25 | 0.25 |
| DCM_{DP} conc., g/g | 0.52 | 0.53 | 0.53 |
| MeOH_{DP} conc., g/g | 0.17 | 0.17 | 0.17 |
| PVA_{CP} conc., g/g | 0.0035 | 0.0035 | 0.0035 |
| Dilution composition | 0 | 0 | 0 |
| Microparticle formation time | 50 sec | 40 min | 70 min |

The properties of the microparticles formed in Table 20 are shown in Table 21.

**Table 21**

| Batch | GC071304 | 0E5103 | 0D4022 |
|---|---|---|---|
| Batch Size, g | 8 | 400 | 700 |
| Microparticle formation duration | 50 sec | 40 min | 70 min |
| Drug Load, % | 14.0 | 13.9 | 12.0 |
| Part. Size Dist., | 4 | 3 | 3 |
| 10% CVF Under, micron | | | |
| Part. Size Dist., 25% CVF Under, micron | 12 | 11 | 11 |
| Part. Size Dist., 50% CVF Under, micron | 27 | 26 | 25 |
| Part. Size Dist., 75% CVF Under, micron | 39 | 39 | 36 |
| Part. Size Dist., 90% CVF Under, micron | 48 | 50 | 45 |
| D. R. (Acc.), 1 Hours | 22.1±1.9 | 15.6 ± 0.8 | 7.7 ± 0.5 |
| D. R. (Acc.), 5 Hours | 41.6±0.5 | 36.2 ± 0.5 | 24.3 ± 0.2 |
| D. R. (Acc.), 25 Hours | 61.6±0.0 | 58.6 ± 0.6 | 48.1± 0.0 |
| D. R. (Acc.), 48 Hours | 75.0±1.1 | 72.5 ± 0.7 | 66.2 ± 0.0 |
| D. R. (Acc.), 72 Hours | 83.8±1.2 | 81.0 ± 0.6 | 78.3 ± 0.2 |
| D. R. (Acc.), 96 Hours | 89.8±1.2 | 86.4 ± 0.6 | 85.3 ± 0.3 |
| D. R. (Phys.), 1 Day | 0.7 | 0.3 | 0.3 |
| D. R. (Phys.), 7 Day | 3.4 | 0.7 | 0.5 |
| D. R. (Phys.), 14 Day | 7.8 | 2.5 | 0.9 |
| D. R. (Phys.), 21 Day | 12.6 | 4.2 | 1.6 |
| D. R. (Phys.), 28 Day | 24.1 | 17.2 | 9.3 |

The release rate, as tested under physiological conditions, were exposed to PBS with a pH of 7.4 at 37°C. The 8 g batch prepared with the shortest microparticle formation time of 50 sec and the shortest corresponding solvent exposure time had fastest initial release, as compared to the 400 g batch and the 700g batch. The largest batch, 700 g, with the longest solvent exposure time (microparticle formation time of 70 minutes) had the slowest initial release. Therefore, under physiological and accelerated conditions, it has been found that increasing the size of the batch, or scaling-up the batch, has an inverse effect on the release rates of the drug, all other factors being approximately the same.

Detailed below in Table 22, microparticles are prepared in 10, 100, and 1000 g scale batches. At 100 g scale, three batches were produced to show that the reproducibility of the examples. The batches were prepared using a 0.183 IV R202H polymer with a molecular weight of 14 kDa, leuprolide, DCM, MeOH, and glacial acetic acid for the dispersed phase and water and polyvinyl alcohol for the continuous phase. Also, approximately 1500, ±500 ml of dilution composition was added to the continuous phase. Specifically, the dilution composition, or water, was added to the solvent removal vessel at 2 to 4 L/min continuously during the microparticle preparation. Preparation parameters are same for the batches and the duration of microparticle preparation was 1 min for 10 g batch, 10 min for 100 g batch and 100 min for the 1000 g batch. At 100g scale, three batches were prepared to show reproducibility.

**Table 22**

| | |
|---|---|
| L.A_{DP} conc., g/g | 0.0435 |
| R202H_{DP} conc., g/g | 0.2087 |
| DCM_{DP} conc., g/g | 0.53 |
| MeOH_{DP} conc., g/g | 0.1920 |
| A. Acid_{DP} conc., g/g | 0.0165 |
| PVA_{CP} conc., g/g | 0.0035 |
| CP Flow rate, g/min | 2000±200 |
| DP Flow, ml/min | 30±3 |
| Dilution composition Added, m | 1500±500 |
| Silverson, rpm | 7000±200 |
| Temperature of wash | Ambient and 39°C |
| Number of Volume replacements | 12±2 |

**Table 23**

| Batch | TV042706 | GC050306 | GC050406 | TV051706 | OL061206 |
|---|---|---|---|---|---|
| Batch Size, g | 10 | 100 | 100 | 100 | 1000 |
| Drug Load, % | 12.8 | 13.4 | 13.2 | 13.1 | 13.7 |
| Drug Encapsulation Efficiency, % | 75 | 77 | 77 | 75 | 79 |
| Part. Size Dist., 10% CVF Under, micron | 3.72 | 3.63 | 3.29 | 3.50 | 3.43 |
| Part. Size Dist., 25% CVF Under, micron | 11.4 | 11.5 | 10.5 | 11.3 | 10.3 |
| Part. Size Dist., 50% CVF Under, micron | 22.2 | 22.5 | 22.0 | 23.6 | 23.4 |
| Part. Size Dist., 75% CVF Under, micron | 30.7 | 31.0 | 30.5 | 32.8 | 33.1 |
| Part. Size Dist., 90% CVF Under, micron | 38.1 | 38.0 | 37.7 | 40.5 | 42.4 |
| D. R. (Acc.), 1 Hours | 27 | 34 | 32 | 30 | 28 |
| D. R. (Acc.), 5 Hours | 48 | 53 | 52 | 51 | 50 |
| D. R. (Acc.), 24 Hours | 75 | 76 | 76 | 74 | 72 |
| D. R. (Acc.), 48 Hours | 87 | 87 | 86 | 86 | 83 |
| D. R. (Acc.), 72 Hours | 92 | 91 | 90 | 91 | 88 |
| Residual DCM, ppm | <100 | <100 | <100 | <100 | <100 |
| Residual MeOH, ppm | <100 | <100 | <100 | <100 | <100 |

The drug encapsulation efficiency is comparable among the 10, 100, and 1000 g batches with values ranging from 75 to 79%. The drug load is between 12.8 and 13.7%, a normal variation. The particle size result shows comparable size distribution data. The drug release rate is similar for 10, 100, and 1000 g batches. Batches made at 10, 100, and 1000 g all showed very low residual solvent. Therefore, by adding a dilution composition to the continuous phase, the size of the batch becomes less of a factor, with regard to the above measured release profiles. Once the solvent level in the continuous phase is reduced beneath a threshold level, the release properties of the microparticles are not as dependant on the size of the batch as were the batches made without the dilution composition added.

### Example 11

Example 11 details the release profiles of risperidone microparticles prepared with a dilution composition. First, microparticles were prepared using 27.5 g of poly(D,L-lactide-co-glycolide), PLGA with a lactide:glycolide ratio of 85:15 and an inherent viscosity of 0.61 g/dL, as the biocompatible and biodegradable polymer. The batches were made using 27.5 g of risperidone. The risperidone and the polymer were dissolved in 220 g of dichloromethane. This combined organic solution of polymer, biologically active agent, and solvent made up the dispersed phase. Separately, 87.5g of polyvinyl alcohol and 69 g of sodium phosphate dibasic heptahydrate were dissolved in 25kg of purified water to provide the continuous phase. The dispersed phase and the continuous phase were pumped simultaneously at 38.5 ml/min and 4000 mL/min respectively, into the Silverson in-line mixer, which was mixed at 4000 rpm for approximately 5 minutes.

As the dispersed phase was mixed with the continuous phase, the resulting mixture solidified into risperidone loaded PLGA microparticles in the Silverson mixer. The suspension of risperidone microparticles was discharged to the solvent removal vessel (SRV) where a dilution composition was added to the microparticle dispersion at room temperature. The dilution composition was added at a rate of 2000 mL/min for approximately 5 minutes. The microparticles were washed and the organic solvents were removed in the SRV using room temperature and 33-37°C water. After the washing and solvent removal, the microparticle were collected by filtration and freeze dried. The microparticles were determined to have a drug load of 0.39 g risperidone per g microparticles. The risperidone loading efficiency in the PLGA microparticles was 77.5%. The release properties of the microparticles were evaluated. It was found that less than 1% of initial release of the drug was detected in the pH 7 buffer within 24 hours.

### Example 12

Example 12 details the release profiles of risperidone encapsulated in microparticles without water dilution. The microparticles were prepared using 27.5 g of poly(D,L-lactide-co-glycolide), PLGA with a lactide:glycolide ratio of 85:15 and an inherent viscosity of 0.61 g/dL. 27.5 g of risperidone was dissolved in 220 g of dichloromethane as part of an organic solution which served as the dispersed phase. Separately, the continuous phase was prepared with 87.5g of polyvinyl alcohol and 69 g of sodium phosphate dibasic heptahydrate dissolved in 25kg of purified water. The dispersed phase and the continuous phase were simultaneously pumped into a Silverson in-line mixer at a 38.5 ml/min and 4000 mL/min, respectively. The combined suspension was mixed at 4000 rpm. The dispersed phase was mixed with the continuous phase and the resulting mixture instantly solidified into risperidone loaded PLGA microparticles in the Silverson mixer. The suspension of risperidone microparticles was discharged to the solvent removal vessel (SRV). No dilution composition was used in this example. The microparticles were washed and the organic solvents were removed in the SRV using room temperature and 33-37°C water. After the washing and solvent removal steps, the microparticles were collected by filtration and freeze dried. The microparticles were determined to have a content of 0.33 g risperidone per g microparticles. The risperidone loading efficiency into the PLGA microparticles was 66.2%. Approximately 80-82% risperidone was released from the microparticles in the pH 7 buffer within 24 hours following testing for release properties.

**Table 24**

| Example | Example 8 | Example 9 |
|---|---|---|
| Polymer | 85:15 PLGA | 85:15 PLGA |
| Target Risperidone, % | 10 | 10 |
| Risperidone_{DP}, g | 27.5 | 27.5 |
| PLGA_{DP}, g | 27.5 | 27.5 |
| DCM_{DP}, g | 220 | 220 |
| MeOH_{DP}, g | 1.02 | 1.02 |
| A.Acid_{DP}, g | 0.78 | 0.78 |
| PVA_{CP} conc., % | 0.35 | 0.35 |
| Sodium Phosphate dibasic heptahydrate_{CP,} % | 0.27 | 0.27 |
| Silverson, rpm | 4000 | 4000 |
| CP Flow Rate, ml/min | 4000 | 4000 |
| DP Flow Rate, ml/min | 38.5 | 38.5 |
| Dilution composition Flow Rate, ml/min | 2000 | N/A |
| Drug Load | 0.39 | 0.33 |
| Drug encapsulation efficiency, % | 77.5 | 66.2 |
| Initial Release, % | <1 | 80-82 |

For the batch made without a dilution composition added, the release rate was 80-82% of risperidone from the microparticle. The batch that was diluted with the dilution composition at 2000 ml/min had a release rate of less than 1% upon initial administration.

## Claims

1. A method for forming a sustained release composition with an altered release rate comprising the steps of:
forming a dispersed phase comprising a specific active agent dissolved with a polymer;
mixing the dispersed phase with an aqueous continuous phase to form a microparticle dispersion comprising microparticles suspended in the continuous phase; and
adding a measured amount of a dilution composition to the microparticle dispersion after the microparticles have been formed, wherein the formed microparticles are sufficiently solid to be filterable using a hollow fiber filter;
wherein the amount of the dilution composition added is sufficient to alter the release rate for the specific active agent.

2. The method of claim 1, wherein the dilution composition is sufficient to dilute the continuous phase to negate adverse effects of increasing a batch size of the sustained release composition.

3. The method of claim 1,
wherein (a) the dilution composition is added to a freshly formed microparticle and is transferred to a first vessel, or
wherein (b) the dilution composition is added to the microparticle dispersion after it is transferred to a first vessel.

4. The method of claim 1,
wherein (a) the method further comprises transferring the microparticle dispersion from a mixer to a first vessel while simultaneously adding the dilution composition to the first vessel, or
wherein (b) the dilution composition is water, and wherein the water is added to the microparticle dispersion in an amount of at least 50% to approximately 300% of the continuous phase, or
wherein (c) the dilution composition and the microparticle dispersion are simultaneously transferred to a first vessel.

5. A method for forming a sustained release composition with an altered release rate comprising the steps of:
forming a dispersed phase comprising a specific active agent and a polymer;
mixing the dispersed phase and an aqueous continuous phase to form a microparticle dispersion comprising microparticles that are sufficiently solid to be filterable using a hollow fiber filter suspended in the continuous phase; and
adding a dilution composition to the microparticle dispersion comprising microparticles that are sufficiently solid to be filterable using a hollow fiber filter to produce a sustained release microparticle having a release rate different than a release rate of an untreated sustained release microparticle;
wherein the volume of the dilution composition is at least 50% of the volume of the continuous phase.

6. The method of claim 5, wherein the specific active agent is leuprolide, the leuprolide is dissolved in the polymer and, wherein the volume of the dilution composition is at least 200% of the volume of the continuous phase, preferably wherein the continuous phase comprises a solvent, and wherein the dilution composition is added to the continuous phase portion until the solvent has a concentration of less than about 5000 ppm of the solvent.

7. The method of claim 5,
wherein (a) the specific active agent is octreotide, and wherein the volume of the dilution composition is at least 100% of the volume of the continuous phase, or
wherein (b) the specific active agent is risperidone.

8. The method of claim 5, wherein the method is performed continuously.

9. The method of claim 5, wherein the microparticles that are sufficiently solid to be filterable using a hollow fiber filter, are formed in about 2 to about 30 seconds after the dispersed phase is mixed with the continuous phase, and wherein the microparticles are exposed to the dilution composition for a measured period of time.

10. The method of claim 5, wherein the dilution composition is sufficient to dilute the continuous phase to negate adverse effects of increasing a batch size of the sustained release composition.

11. A method for controlling a release rate of a sustained release composition comprising the steps of:
forming the dispersed phase comprising the specific active agent and the polymer, wherein the specific active agent is leuprolide and the leuprolide is dissolved in the polymer,
mixing the dispersed phase with the continuous phase in a mixer to form the microparticle dispersion;
adding a measured amount of the dilution composition to the microparticle dispersion after microparticles have been formed, wherein the formed microparticles are sufficiently solid to be filterable using a hollow fiber filter;
wherein the amount of the dilution composition is sufficient to alter the release rate for the leuprolide;
and wherein the volume of the dilution composition is at least 50% of the volume of the continuous phase.

12. The method of claim 11, wherein the continuous phase comprises a solvent and the amount of the dilution composition added is sufficient to dilute the solvent in the continuous phase to less than about 5000 ppm.

13. The method of claim 11, wherein the microparticle dispersion is maintained in a mixer for a defined period of time before being transferred to a first vessel.

## Patentansprüche

1. Verfahren zur Herstellung einer Zusammensetzung mit einer verzögerten Freisetzung und mit einer veränderten Freisetzungsrate, welches die Schritte aufweist:
ein Bilden einer dispergierten Phase, die einen spezifischen Wirkstoff aufweist, der mit einem Polymer gelöst ist,
ein Mischen der dispergierten Phase mit einer wässrigen kontinuierlichen Phase, um eine Mikropartikeldispersion zu bilden, die Mikropartikel aufweist, die in der kontinuierlichen Phase suspendiert sind, und
ein Hinzufügen einer gemessenen Menge einer Verdünnungszusammensetzung zu der Mikropartikeldispersion, nachdem die Mikropartikel gebildet worden sind, wobei die gebildeten Mikropartikel ausreichend fest sind, um unter Verwendung eines Hohlfaserfilters filtrierbar zu sein,
wobei die Menge der hinzugefügten Verdünnungszusammensetzung ausreichend ist, um die Freisetzungsrate für den spezifischen Wirkstoff zu verändern.

2. Verfahren nach Anspruch 1, wobei die Verdünnungszusammensetzung ausreicht, um die kontinuierliche Phase zu verdünnen, um nachteilige Effekte der Erhöhung der Chargengröße der Zusammensetzung mit einer verzögerten Freisetzung zu verhindern.

3. Verfahren nach Anspruch 1,
wobei (a) die Verdünnungszusammensetzung einem frisch gebildeten Mikropartikel hinzugefügt wird und in ein erstes Gefäß übertragen wird oder
wobei (b) die Verdünnungszusammensetzung der Mikropartikeldispersion hinzugefügt wird, nachdem sie in ein erstes Gefäß übertragen worden ist.

4. Verfahren nach Anspruch 1,
wobei (a) das Verfahren ferner ein Übertragen der Mikropartikeldispersion von einem Mischer zu einem ersten Gefäß aufweist, während gleichzeitig die Verdünnungszusammensetzung dem ersten Gefäß hinzugefügt wird, oder
wobei (b) die Verdünnungszusammensetzung Wasser ist und wobei das Wasser der Mikropartikeldispersion in einer Menge von mindestens 50 % bis etwa 300 % der kontinuierlichen Phase hinzugefügt wird oder
wobei (c) die Verdünnungszusammensetzung und die Mikropartikeldispersion gleichzeitig in ein erstes Gefäß übertragen werden.

5. Verfahren zur Herstellung einer Zusammensetzung mit einer verzögerten Freisetzung und mit einer veränderten Freisetzungsrate, welches die Schritte aufweist:
ein Bilden einer dispergierten Phase, die einen spezifischen Wirkstoff und ein Polymer aufweist,
ein Mischen der dispergierten Phase und einer wässrigen kontinuierlichen Phase, um eine Mikropartikeldispersion zu bilden, die Mikropartikel aufweist, die ausreichend fest sind, um bei einer Verwendung eines Hohlfaserfilters filtrierbar zu sein und die in der kontinuierlichen Phase suspendiert sind, und
ein Hinzufügen einer Verdünnungszusammensetzung zu der Mikropartikeldispersion, die Mikropartikel aufweist, die ausreichend fest sind, um bei einer Verwendung eines Hohlfaserfilters filtrierbar zu sein, um ein Mikropartikel mit einer verzögerten Freisetzung zu erzeugen, das eine Freisetzungsrate aufweist, die sich von der Freisetzungsrate eines unbehandelten Mikropartikels mit einer verzögerten Freisetzung unterscheidet,
wobei das Volumen der Verdünnungszusammensetzung mindestens 50 % des Volumens der kontinuierlichen Phase beträgt.

6. Verfahren nach Anspruch 5, wobei der spezifische Wirkstoff ein Leuprolid ist, wobei das Leuprolid in dem Polymer gelöst ist und wobei das Volumen der Verdünnungszusammensetzung mindestens 200 % des Volumens der kontinuierlichen Phase beträgt, wobei bevorzugt die kontinuierliche Phase ein Lösungsmittel aufweist und wobei die Verdünnungszusammensetzung der Portion der kontinuierlichen Phase hinzugefügt wird, bis das Lösungsmittel eine Konzentration von weniger als etwa 5000 ppm des Lösungsmittels aufweist.

7. Verfahren nach Anspruch 5,
wobei (a) der spezifische Wirkstoff ein Octreotid ist und wobei das Volumen der Verdünnungszusammensetzung mindestens 100 % des Volumens der kontinuierlichen Phase beträgt oder
wobei (b) der spezifische Wirkstoff ein Risperidon ist.

8. Verfahren nach Anspruch 5, dadurch wobei das Verfahren kontinuierlich durchgeführt wird.

9. Verfahren nach Anspruch 5, wobei die Mikropartikel, die ausreichend fest sind, um bei einer Verwendung eines Hohlfaserfilters filtrierbar zu sein, in etwa 2 bis etwa 30 Sekunden nach der Vermischung der dispergierten Phase mit der kontinuierlichen Phase gebildet werden und wobei die Mikropartikel der Verdünnungszusammensetzung für einen gemessenen Zeitraum ausgesetzt werden.

10. Verfahren nach Anspruch 5, wobei die Verdünnungszusammensetzung ausreichend ist, um die kontinuierliche Phase zu verdünnen, um nachteilige Effekte der Erhöhung der Chargengröße der Zusammensetzung mit einer verzögerten Freisetzung zu verhindern.

11. Verfahren zum Steuern einer Freisetzungsrate einer Zusammensetzung mit einer verzögerten Freisetzung, welches die Schritte aufweist:
ein Bilden der dispergierten Phase, die den spezifischen Wirkstoff und das Polymer aufweist, wobei der spezifische Wirkstoff ein Leuprolid ist und wobei das Leuprolid in dem Polymer gelöst ist,
ein Mischen der dispergierten Phase mit der kontinuierlichen Phase in einem Mischer zur Bildung der Mikropartikeldispersion,
ein Hinzufügen einer gemessenen Menge der Verdünnungszusammensetzung zu der Mikropartikeldispersion, nachdem die Mikropartikel gebildet worden sind, wobei die gebildeten Mikropartikel ausreichend fest sind, um bei einer Verwendung eines Hohlfaserfilters filtrierbar zu sein,
wobei die Menge der Verdünnungszusammensetzung ausreichend ist, um die Freisetzungsrate für das Leuprolid zu verändern, und
wobei das Volumen der Verdünnungszusammensetzung mindestens 50 % des Volumens der kontinuierlichen Phase beträgt.

12. Verfahren nach Anspruch 11, wobei die kontinuierliche Phase ein Lösungsmittel aufweist und die Menge der hinzugefügten Verdünnungszusammensetzung ausreichend ist, um das Lösungsmittel in der kontinuierlichen Phase auf weniger als etwa 5000 ppm zu verdünnen.

13. Verfahren nach Anspruch 11, wobei die Mikropartikeldispersion für einen definierten Zeitraum in einem Mischer gehalten wird, bevor sie in ein erstes Gefäß übertragen wird.

## Revendications

1. Procédé de formation d'une composition à libération prolongée avec une vitesse de libération modifiée comprenant les étapes consistant à :
former une phase dispersée comprenant un principe actif spécifique dissous avec un polymère ;
mélanger la phase dispersée avec une phase continue aqueuse pour former une dispersion de microparticules comprenant des microparticules suspendues dans la phase continue ; et
ajouter une quantité mesurée d'une composition de dilution à la dispersion de microparticules après la formation des microparticules, les microparticules formées étant suffisamment solides pour pouvoir être filtrées en utilisant un filtre à fibres creuses ;
dans lequel la quantité de la composition de dilution ajoutée étant suffisante pour modifier la vitesse de libération du principe actif spécifique.

2. Procédé selon la revendication 1, dans lequel la composition de dilution est suffisante pour diluer la phase continue afin de supprimer les effets indésirables de l'augmentation de la taille d'un lot de la composition à libération prolongée.

3. Procédé selon la revendication 1,
dans lequel (a) la composition de dilution est ajoutée à une microparticule fraichement formée et est transférée dans un premier récipient, ou
dans lequel (b) la composition de dilution est ajoutée à la dispersion de microparticules après son transfert dans un premier récipient.

4. Procédé selon la revendication 1,
dans lequel (a) le procédé comprend en outre le transfert de la dispersion de microparticules d'un mixeur dans un premier récipient tout en ajoutant simultanément la composition de dilution dans le premier récipient, ou
dans lequel (b) la composition de dilution est de l'eau et dans lequel l'eau est ajoutée à la dispersion de microparticules en une quantité d'au moins 50 % à environ 300 % de la phase continue, ou
dans lequel (c) la composition de dilution et la dispersion de microparticules sont transférées simultanément dans un premier récipient.

5. Procédé de formation d'une composition à libération prolongée avec une vitesse de libération modifiée comprenant les étapes consistant à :
former une phase dispersée comprenant un principe actif spécifique et un polymère ;
mélanger la phase dispersée et une phase continue aqueuse pour former une dispersion de microparticules comprenant des microparticules qui sont suffisamment solides pour pouvoir être filtrées en utilisant un filtre à fibres creuses suspendue dans la phase continue ; et
ajouter une composition de dilution à la dispersion de microparticules comprenant des microparticules qui sont suffisamment solides pour pouvoir être filtrées en utilisant un filtre à fibres creuses pour produire une microparticule à libération prolongée ayant une vitesse de libération différente de la vitesse de libération d'une microparticule à libération prolongée non traitée ;
le volume de la composition de dilution étant au moins 50 % du volume de la phase continue.

6. Procédé selon la revendication 5, dans lequel le principe actif spécifique est le leuprolide, le leuprolide est dissous dans le polymère, et dans lequel le volume de la composition de dilution est au moins 200 % du volume de la phase continue, de préférence dans lequel la phase continue comprend un solvant, et dans lequel la composition de dilution est ajoutée à la partie de phase continue jusqu'à ce que le solvant ait une concentration inférieure à environ 5 000 ppm de solvant.

7. Procédé selon la revendication 5,
dans lequel (a) le principe actif spécifique est l'octréotide, et dans lequel le volume de la composition de dilution est au moins 100 % du volume de la phase continue, ou
dans lequel (b) le principe actif spécifique est la rispéridone.

8. Procédé selon la revendication 5, le procédé étant réalisé de manière continue.

9. Procédé selon la revendication 5, dans lequel les microparticules qui sont suffisamment solides pour pouvoir être filtrées en utilisant un filtre à fibres creuses sont formées en environ 2 à environ 30 secondes après le mélange de la phase dispersée avec la phase continue, et dans lequel les microparticules sont exposées à la composition de dilution pendant une période de temps mesurée.

10. Procédé selon la revendication 5, dans lequel la composition de dilution est suffisante pour diluer la phase continue afin de supprimer les effets indésirables de l'augmentation de la taille d'un lot de la composition à libération prolongée.

11. Procédé de régulation de la vitesse de libération d'une composition à libération prolongée comprenant les étapes consistant à :
former la phase dispersée comprenant le principe actif spécifique et le polymère, le principe actif spécifique étant le leuprolide et le leuprolide étant dissout dans le polymère,
mélanger la phase dispersée avec la phase continue dans un mixeur pour former la dispersion de microparticules ;
ajouter une quantité mesurée de la composition de dilution à la dispersion de microparticules après la formation des microparticules, les microparticules formées étant suffisamment solides pour pouvoir être filtrées en utilisant un filtre à fibres creuses ;
la quantité de la composition de dilution étant suffisante pour modifier la vitesse de libération du leuprolide,
et le volume de la composition de dilution étant au moins 50 % du volume de la phase continue.

12. Procédé selon la revendication 11, dans lequel la phase continue comprend un solvant et la quantité de la composition de dilution ajoutée est suffisante pour diluer le solvant dans la phase aqueuse à moins d'environ 5 000 ppm.

13. Procédé selon la revendication 11, dans lequel la dispersion de microparticules est maintenue dans un mixeur pendant une période de temps définie avant d'être transférée dans un premier récipient.
